# EUROPEAN PATENT SPECIFICATION

(11) **EP 1 601 679 B1**
(45) Date of publication and mention of the grant of the patent: **11.05.2011**
(21) Application number: 04717576.5
(22) Date of filing: 05.03.2004
(51) Int. Cl.: C07F 9/09, A61K 6/08, C08F 30/02

(54) **A POYMERIZABLE PHOSPHORIC ACID ESTER DERIVATIVE AND A DENTAL COMPOSITION EMPLOYING IT.**
POLYMERISIERBARE PHOSPHORSÄUREESTER-DERIVATE UND DIESE ENTHALTENDES DENTALMATERIAL
DERIVES POLYMERISABLES D'ESTERS D'ACIDE PHOSPHORIQUE ET COMPOSITION DENTAIRE LES CONTENANT

(30) Priority: 07.03.2003 EP 03005174
(43) Date of publication of application: 07.12.2005
(73) Proprietor: DENTSPLY DETREY GmbH, 78467 Konstanz (DE)
(72) Inventor: KLEE, Joachim, E., 78315 Radolfzell (DE); LEHMANN, Uwe, 78467 Konstanz (DE); WALZ, Uwe, 78465 Konstanz (DE); LIU, Huaibing, Dover, DE 19901 (US)
(74) Representative: Hartz, Nikolai
(86) International application number: PCT/EP2004/002289
(87) International publication number: WO 2004/078100

(56) References cited:
- EP-A- 1 148 060
- EP-A- 1 169 996
- WO-A-00/10478
- DE-A- 19 746 708
- US-A- 4 539 382
- MOSZNER N ET AL: "MONOMERS FOR ADHESIVE POLYMERS, 2 SYNTHESIS AND RADICAL POLYMERISATION OF HYDROLYTICALLY STABLE ACRYLIC PHOSPHONIC ACIDS" MACROMOLECULAR CHEMISTRY AND PHYSICS, WILEY VCH, WEINHEIM, DE, vol. 200, no. 5, May 1999 (1999-05), pages 1062-1067, XP000847445 ISSN: 1022-1352 cited in the application

## Description

The present invention relates to a polymerizable phosphoric acid ester derivative, its use in a dental composition, a process for its production, and a dental composition employing it. More specifically the polymerizable phosphoric acid ester derivative, due to its stability against hydrolysis in acidic medium, allows the provision of a one-part self-etching, self-priming dental adhesive composition.

### Technical background

WO 00/10478 discloses an adhesive composition comprising an unsaturated phosphate ester. Presently, self-etching, self-priming dental adhesives are composed of two-part systems due to low hydrolysis stability of the polymerizable acidic monomers. The low hydrolysis stability arises from the hydrolysis of acidic and adhesive monomers in water or water/solvent mixtures. Therefore, the known acidic and adhesive monomers must be stored water-free and mixed with the aqueous part just before application.

Frequently, sulfonic acid and phosphoric acid ester groups are employed in acidic polymerizable adhesive monomers. However, these acidic groups hydrolyze the acrylic and methacrylic ester moieties as well as the phosphoric acid ester groups within the monomers (Moszner et al. Macromol. Chem. Phys. 200, 1062, (1999), DE 199 18 974, EP 1 169 996). In order to overcome these disadvantages, polymerizable phosphonic acid monomers were proposed by Moszner et al. Macromol. Chem. Phys. 200, 1062, (1999), DE 199 18 974, and EP 1 169 996. Moreover, US 4,539,382 discloses mono(meth)acrylamides with one phosphonic acid group. However, these monomers still comprise hydrolysable (meth)acrylic ester moieties. Therefore, monomers with phosphonic acid ester groups based on 2-(oxa alkyl) acrylate were suggested in DE 197 46 708. However, also these phosphonic acid derivatives tend to hydrolyse in acidic solution. Therefore, it has not been possible to provide an one-part self-etching, self-priming dental adhesive composition. An one-part composition means that the composition is contained in only one container which may be stored. It allows application of the composition without any mixing and without any special equipment before the application. Self-etching means that the dental adhesive composition may be applied to a tooth without any preliminarily etching of enamel in a separate method step. In order to comprise such a self-etching feature, the composition must be acidic. Self-priming means that the dental adhesive composition may be applied to a tooth without any preliminarily application of a primer.

In practice the monomers of the prior art could be employed only in two-part dental systems which consist of a priming part and a bonding part. These two-part dental adhesive systems are either applied sequentially or in one step after mixing the two parts. Both procedures have inherent disadvantages due to clinical complications which might occur between sequential steps (saliva or blood contamination) or due to dosing problems when mixing is required prior to the application of the self-etching adhesive.

In order to overcome these clinical problems it is desired to provide a self-priming and self-etching adhesive as a one-part system eliminating the need of sequential application or premixing.

Further disadvantages of the monomers of the prior art containing phosphonic acid derivatives are as follows: The phosphonic acids are less acidic than phosphoric acid. Therefore, additional acids are required for obtaining the self-etching feature of a dental composition. However, the additional acid increases generally degradation of the monomer by hydrolysis. Moreover, the intermediates for producing the phosphonic acid derivatives are toxic. Therefore, the process for the preparation is dangerous and more complicated. Further, the phosphonic acid derivatives are more expensive than phosphoric acid derivatives.

### Description of the invention

The above described needs and the disadvantages of the polymerizable phosphonic acid derivatives are solved by the polymerizable phosphoric acid ester derivative having the following formula (A): 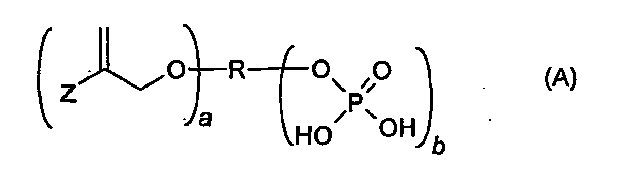 wherein
Z is COOR₁, COSR₁, CON(R₁)₂, CONR₁R₂, or CONHR₁,
R₁ and R₂ independently are a hydrogen atom, a substituted or unsubstituted C₁ to C₁₈ alkyl group optionally substituted by a C₃ to C₈ cycloalkyl group, a substituted or unsubstituted C₃ to C₈ cycloalkyl group, a substituted or unsubstituted C₄ to C₁₈ aryl or heteroaryl group, a substituted or unsubstituted C₅ to C₁₈ alkylaryl or alkylheteroaryl group, or a substituted or unsubstituted C₇ to C₃₀ aralkyl group, whereby two R₁ residues may form together with the N-atom to which they are bonded a 5- to 7-membered heterocyclic ring which may contain beside said N-atom a further nitrogen atom or an oxygen atom, and whereby the substituted groups may be substituted by 1 to 5 C₁ to C₅ alkyl group(s);
a is an integer of from 1 to 10, preferably 1 to 5;
b is an integer of from 1 to 10, preferably 1 to 5; and
R represents an organic residue as further defined by claim 1.

In this specification an aryl group may for example be a phenyl group, naphthyl group, anthryl group, etc., whereby a phenyl group representing a C₆ aryl group is a preferred aryl group. A heteroaryl group may for example be a furyl or thienyl group representing a C₄ heteroaryl group, whereby a thienyl group is particularly preferred.

The polymerizable phosphoric acid ester derivative of the present invention has surprisingly high hydrolysis stability, although a phosphate group is present. Surprisingly, it is hydrolysis stable both at the 2-(oxa-ethyl)-group and at the phosphoric acid ester group. It is hydrolysis stable under acidic conditions. Particularly, it is hydrolysis stable at a pH of at most 4, preferably at a pH of at most 2, most preferably at a pH of 1.0. Therefore, the polymerizable phosphoric acid ester derivative of the present invention allows the preparation of an advantageous one-pack self-etching and self-priming dental adhesive composition.

A one-pack composition means that the composition of the present invention is contained in only one container which may be stored and allows application of the composition without any mixing and without any special equipment before the application.

Self-etching means that the dental adhesive composition of the present invention may be applied to a tooth without any preliminarily etching of enamel in a separate method step. Particularly, the polymerizable phosphoric acid ester derivative of the present invention allows the preparation of a dental composition which is hydrolysis stable for at least one week at a storage temperature of 50 °C, whereby after such storage the bond strength of an adhesive prepared from such a dental composition to enamel and/or dentin is at least 10 MPa, preferably 15 MPa. Due to the high hydrolysis stability of the compound of the present invention a one-part self-etching and self-priming system which has excellent shelf-life may be prepared.

Further advantages of the polymerizable phosphoric acid ester derivative of the present invention are as follows: the phosphoric acid derivatives are stronger acidic than phosphonic acid derivatives. Therefore, it is not necessary to incorporate additional acids for obtaining the self-etching feature of a dental composition. However, a dental composition according to the present invention may include also further acids. They allow an easy adjustment of the pH. Surprisingly, neither the stronger phosphoric acid derivative of the invention nor additional acid(s) decrease the hydrolysis stability. Moreover, the intermediates for producing the phosphoric acid derivatives are not toxic. Therefore, the process for the preparation is safe, and the process for preparing the polymerizable phosphoric acid ester derivative of the present invention can be conducted more easily. Further, the phosphoric ester derivatives are generally less expensive than phosphonic acid derivatives.

According to the invention the organic residue R is an (*a*+*b*)-valent saturated aliphatic C₂ to C₁₈ group having at least 2 of said primary aliphatic carbon atoms, and optionally 1 or more of said secondary aliphatic carbon atom(s), whereby said (a+b)-valent group may be substituted by C₁ to C₅ alkyl group(s); or a C₂ to C₄₅ mono-, di-; or polyether which has from 1 to 14 oxygen atoms and is substituted by at least 2 C₁ to C₁₀ aliphatic group(s) having said primary and/or secondary aliphatic carbon atoms; whereby said ether may optionally be substituted by C₁ to C₅ alkyl group(s). The amount of these C₁ to C₅ alkyl group(s) may vary. Preferably, the ether may be substituted by 1 to 15, more preferably 1 to 5 C₁ to C₅ alkyl group(s).

According to a further embodiment of the present invention, the organic residue R represents
a saturated C₃ to C₈ cyclic, C₇ to C₁₅ bi- or polycyclic hydrocarbon group having from 0 to 4, preferably, 0 to 3, more preferably 0 or 1, of said secondary alicyclic carbon atoms; and/or
a C₄ to C₁₈ aryl or heteroaryl group having from 0 to 5, preferably 0 to 3, more preferably 0 or 1, of said aromatic carbon atoms;
whereby said saturated hydrocarbon or aryl or heteroaryl group is substituted by from 0 to 5 C₁ to C₅ alkyl group(s);
from 0 to 4, preferably 1 to 3, more preferably 1 or 2, saturated C₁ to C₁₀ aliphatic group(s) having said primary and/or secondary aliphatic carbon atoms, and/or
from 0 to 2 divalent residues according to one of the following formulas:
-[O-CH₂CH₂-]_{f}-wherein f is an integer of from 1 to 10, preferably 1 to 5; -[-O-CH₂CH₂ CH₂-]_{g}- wherein g is an integer of from 1 to 10, preferably 1 to 5; -[O-R₁₂]ₕ- wherein R₁₂ is -CH(CH₃)-CH₂ or -CH₂-CH(CH₃)- and h is an integer of from 1 to 10, preferably 1 to 5;
-[-O-R₁₄]ᵢ-[O-R₁₅]ⱼ- or -[O-R₁₅]ₖ-[O-R₁₄]ₗ- wherein R₁₄ is -CH₂CH₂-, R₁₅ is - CH(CH₃)-CH₂- or -CH₂-CH(CH₃)-, i, j, k, and I are integers whereby 2i+3j ≤ 15 and 2k + 3l ≤ 15,
-[O-CH₂CH₂CH₂CH₂-]ᵣ- wherein r is an integer of 1 or 2;
wherein said divalent residues have one of said primary aliphatic carbon atoms; and
whereby 2 groups selected from said saturated hydrocarbon, aryl, and heteroaryl groups may optionally be linked by a single bond, an alkylene group, or -O-. Said alkylene group may be a C₁ to C₈ alkylene group. Preferably it is a C₁ to C₃ alkylene group, whereby an iso-propylene group is particularly preferred.

In a further embodiment of the present invention the organic residue R is
an (a+b)-valent saturated C₃ to C₈ cyclic or C₇ to C₁₅ bi- or tricyclic hydrocarbon group having at least 2 of said secondary alicyclic carbon atoms;
an (a+b)-valent saturated C₅ to C₁₈ aryl or heteroaryl group having from 2 to 6 of said aromatic carbon atoms;
an (a+b)-valent C₆ to C₁₈ alkylaryl or alkyl heteroaryl group having at least one of said aromatic carbon atoms, at least one of said secondary aliphatic carbon atoms, and optionally one of said primary aliphatic carbon atoms at the terminal end of the alkyl moiety of said alkylaryl or alkylheteroaryl group; or
an (a+b)-valent C₈ to C₃₀ aralkyl group having at least one of said primary aliphatic carbon atoms and at least one of said secondary aliphatic carbon atoms.

Particularly preferred is the polymerizable phosphoric acid ester derivative which has one of the following formulas: 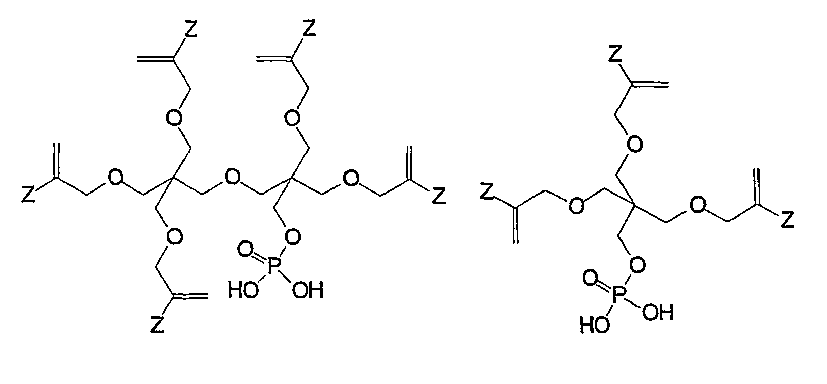 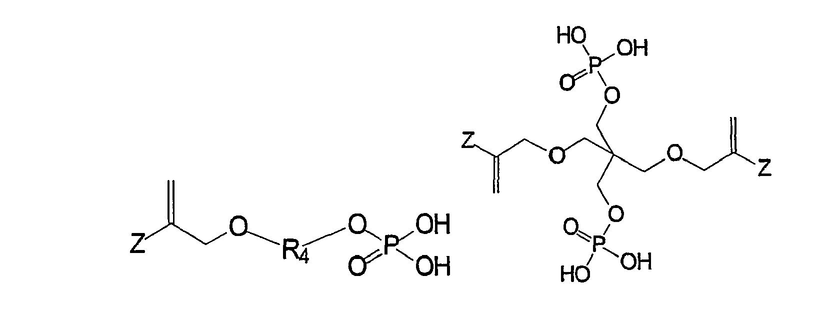 wherein
Z is as defined above,
R₄ denotes a divalent C₂ to C₁₈ alkylene group, a divalent C₃ to C₈ cycloalkylene group, a divalent C₄ to C₁₈ aryl or heteroaryl group, a divalent C₅ to C₁₈ alkylaryl or alkylheteroaryl group, a divalent C₇ to C₃₀ aralkyl group, whereby said groups may be substituted by 1 to 5 C₁ to C₅ alkyl group(s).

The polymerizable phosphoric acid ester derivatives in the above formulas have the advantage that a large number of polymerizable 2-(oxa-ethyl)acryl derivative groups and/or a large number of acidic phosphate groups are linked to one molecule. This allows to tailor the self-priming and self-etching features of a dental composition comprising such compounds. A large amount of polymerizable 2-(oxa-ethyl)acryl derivative groups per molecule enhances the bond strength of a dental adhesive composition comprising the polymerizable phosphoric acid ester derivative of the present invention. A large amount of phosphate groups per molecule enhances the self-etching feature of a dental composition comprising the polymerizable phosphoric acid ester derivative of the present invention.

In a further embodiment of the present invention, the polymerizable phosphoric acid ester derivative has the above formula wherein R₄ is a divalent residue according to one of the following formulas:
-[CH₂CH₂-O-]ₘ-CH₂CH₂- wherein m is an integer of from 1 to 14,
-[CH₂CH₂ CH₂-O-]ₚ-CH₂CH₂ CH₂- wherein p is an integer of from 1 to 14,
-[R₁₂-O]_{q}-R₁₃- wherein R₁₂ and R₁₃ may be -CH(CH₃)-CH₂ or
-CH₂-CH(CH₃)- and q is from 1 to 14,
-[R₁₄-O]ᵣ- [R₁₅-O]ₛ-R₁₄- or -[R₁₄-O]ₜ-[R₁₅-O]ᵤ-R₁₅- wherein R₁₄ is
-CH₂CH₂-, R₁₅ is -CH(CH₃)-CH₂- or -CH₂-CH(CH₃)-, r, s, t, and u are integers whereby 2r + 3s ≤ 43 and 2t + 3u ≤ 42,
-[CH₂CH₂CH₂CH₂-O-]ᵣ-CH₂CH₂CH₂CH₂- wherein r is 1 or 2, 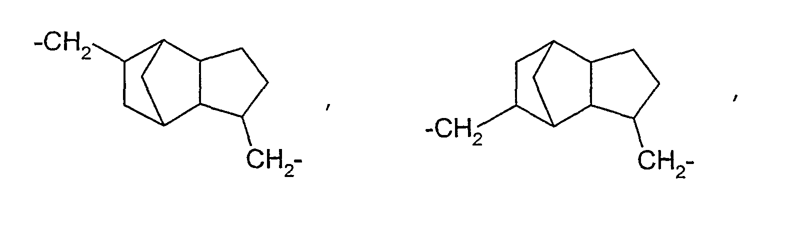 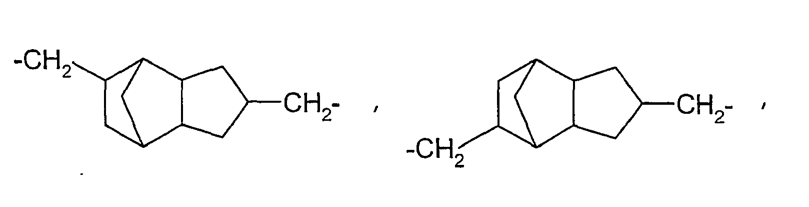 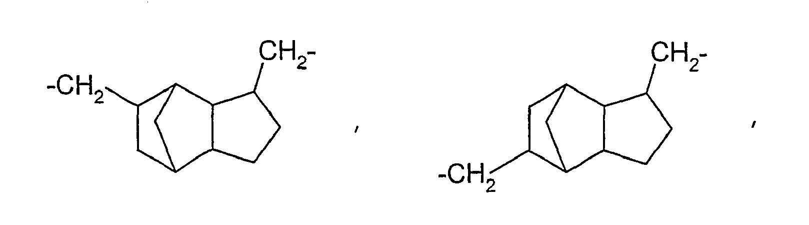 or 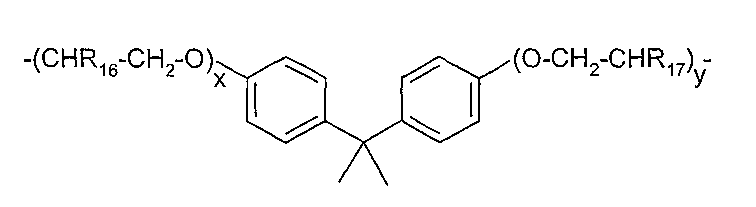 wherein R₁₆ and R₁₇ are H or -CH₃ and x and y may independently be integers of from 0 to 10, preferably 0 to 5.

Particularly preferred is the polymerizable phosphoric acid ester derivative as defined above, wherein said (a+b) carbon atoms are primary aliphatic carbon atoms.

The polymerizable phosphoric acid ester derivative of the present invention is hydrolysis stable under acidic conditions, preferably at a pH of at most 4, more preferably at a pH of at most 2, and most preferably at a pH of 1.0.

The polymerizable phosphoric acid ester derivative of the present invention may be prepared by a process which comprises the following steps:
(i) reacting a di- or polyol of the formula (HO)ₐ-R-(OH)_{b} (B) with a compound of the formula (C): 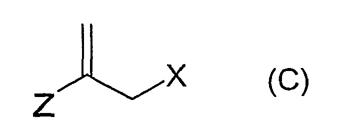 wherein Z, R, a, and b are as defined above, particularly wherein R is R₄ as defined above, and
   X is a leaving group for producing a compound (D) having b hydroxyl group(s) per molecule, and
(ii) reacting compound (D) with a phosphoric acid derivative (E) reactive with a hydroxyl group.

Preferably, the leaving group X is a halogen atom. Particularly preferred is that X is a chlorine or bromine atom, whereby a chlorine atom is most preferred.

The equivalent ratio between compound (C) and the di- or polyol (B) in the above process may be about a : 1. Further, the equivalent ratio between compounds (E) and (D) may be about b : 1. Preferably, the phosphoric acid derivative (E) is phosphorus trichloride oxide. Moreover, the above described method may comprise hydrolyzing the reaction product of compounds (D) and (E).

In case that a polyol is used as compound (B), it may be advantageous that b hydroxyl groups of the polyol (B) are protected, then reacted with compound (C) followed by deprotecting before conducting step (ii). Any known protection agent for protecting hydroxyl groups may be used. Particularly preferred is 3,4-dihydro-2*H*-pyrane, since it is suitable for primary, secondary and aromatic hydroxyl groups. Reacting 3,4-dihydro-2*H*-pyrane with an alcohol under acidic conditions results is a tetrahydropyranylether which is stable under basic conditions and may be cleaved or deprotected easily with mild acids.

Due to the excellent properties of the polymerizable phosphoric acid ester derivative it may be used in a dental composition. Such dental composition includes an adhesive, a primer, a cement, a composite, etc. Particular preferred is a one-part self-etching, self-priming dental adhesive composition.

Further, the present invention provides a dental composition comprising the polymerizable phosphoric acid ester derivative as described above.

In a preferred embodiment of the present invention, the dental composition is acidic. It may have a pH of at most 4, preferably a pH of at most 2, more preferably a pH of about 1.0.

Moreover the dental composition of the present invention may further comprise a curing system. Such a curing system may comprise a polymerization initiator, an inhibitor and a stabilizer. The polymerization initiator may be a thermal initiator, a redox-initiator or a photo initiator. Preferably, camphor quinone is used. A stabilizer may be applied in order to stabilize the dental composition. Such a stabilizer may for example be a radical absorbing monomer, such as hydroquinone, hydroquinone monomethylether, 2,6-di-tert-butyl-p-cresol, tetramethyl piperidine N-oxyl radical, galvanoxyl radical.

In a preferred embodiment of the invention the dental composition may comprise a filler. This filler may be an inorganic filler and/or an organic filler. Preferably, the filler is a nanofiller.

Further, the dental composition of the present invention may comprise an organic water soluble solvent and/or water. The organic water soluble solvent may be selected from alcohols, such as ethanol, propanol, butanol; and/or ketones such as acetone and methyl ethyl ketone. Particularly preferred is acetone, ethanol and/or tert-butanol.

According to a further embodiment of the present invention, the dental composition may comprise an organic and/or inorganic acid. Preferably, the organic acid is a mono- or polycarboxylic acid, such as methacrylic acid, acrylic acid, fumaric acid, maleic acid, citric acid, itaconic acid, and/or formic acid; and the inorganic acid is preferably phosphoric acid, sulfuric acid, a sulfonic acid and/or hydrofluoric acid.

The dental composition of the present invention may also comprise a polymerizable N-substituted alkylacrylic or acrylic acid amide monomer. Preferably, the polymerizable N-substituted alkylacrylic or acrylic acid amide monomer has one of the following formulas: 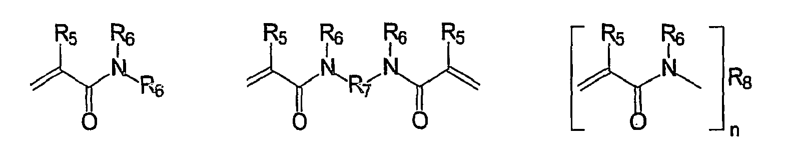 wherein
R₅ and R₆ independently represent an hydrogen atom, a substituted or unsubstituted C₁ to C₁₈ alkyl group, a substituted or unsubstituted C₃ to C₁₈ cycloalkyl group, a substituted or unsubstituted C₄ to C₁₈ aryl or heteroaryl group, a substituted or unsubstituted C₅ to C₁₈ alkylaryl or alkylheteroaryl group, a substituted or unsubstituted C₇ to C₃₀ aralkyl group, whereby two R₆ residues may form together with the N-atom to which they are bonded a 5- to 7-membered heterocyclic ring which may contain beside said N-atom a further nitrogen atom or an oxygen atom, and whereby the substituted groups may be substituted by 1 to 5 C₁ to C₅ alkyl group(s);
R₇ denotes a divalent substituted or unsubstituted organic residue having from 1 to 45 carbon atoms, whereby said organic residue may contain from 1 to 14 oxygen and/or nitrogen atoms and is selected from
a C₁ to C₁₈ alkylene group wherein from 1 to 6 -CH₂-groups may be replaced by a -N-(C=O)-CR₉=CH₂ group wherein R₉ is a hydrogen atom or a C₁ to C₁₈ alkyl group,
a divalent substituted or unsubstituted C₃ to C₁₈ cycloalkyl or cycloalkylene group,
a divalent substituted or unsubstituted C₄ to C₁₈ aryl or heteroaryl group,
a divalent substituted or unsubstituted C₅ to C₁₈ alkylaryl or alkylheteroaryl group,
a divalent substituted or unsubstituted C₇ to C₃₀ aralkyl group, and
a divalent substituted or unsubstituted C₂ to C₄₅ mono-, di- or polyether group having from 1 to 14 oxygen atoms,
R₈ denotes a saturated di- or multivalent substituted or unsubstituted C₂ to C₁₈ hydrocarbon group, a saturated di- or multivalent substituted or unsubstituted cyclic C₃ to C₁₈ hydrocarbon group, a di- or multivalent substituted or unsubstituted C₄ to C₁₈ aryl or heteroaryl group, a di- or multivalent substituted or unsubstituted C₅ to C₁₈ alkylaryl or alkylheteroaryl group, a di- or multivalent substituted or unsubstituted C₇ to C₃₀ aralkyl group, or a di- or multivalent substituted or unsubstituted C₂ to C₄₅ mono-, di-, or polyether residue having from 1 to 14 oxygen atoms,
n is an integer, preferably from 2 to 10, more preferably from 3 to 4.

More preferably, the dental composition of the present invention contains a mono-, bis- or poly(meth) acrylamide monomer characterized by one of the following formulas:

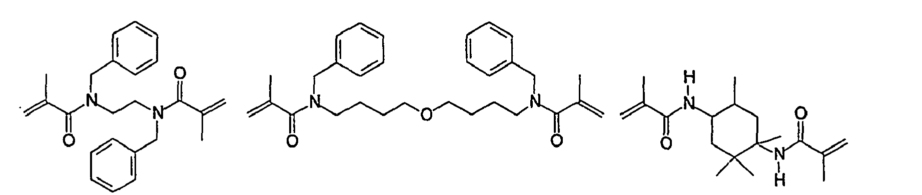

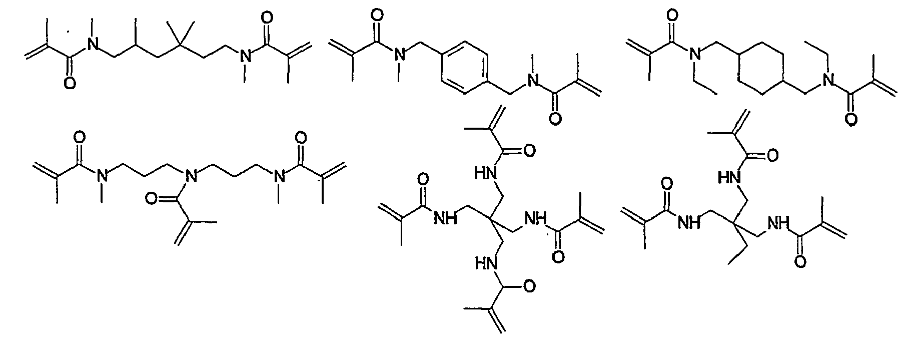

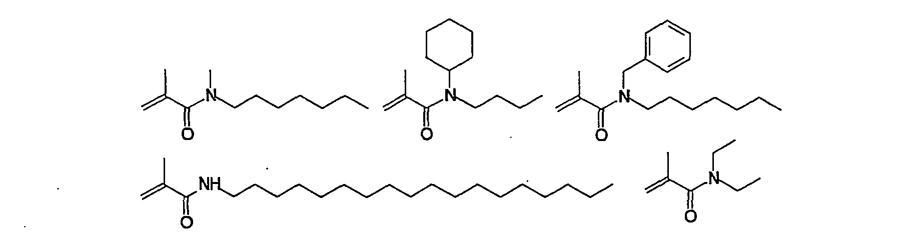

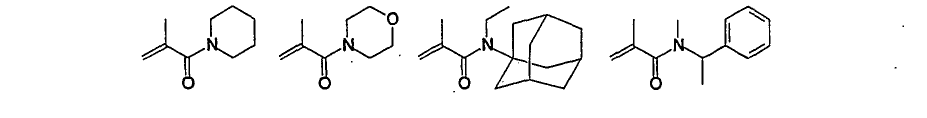

According to a particular preferred embodiment of the present invention, the dental composition is a hydrolysis stable one-part self-etching, self-priming dental adhesive composition. Such a composition is advantageously hydrolysis stable, e.g. for at least one week at a storage temperature of 50 °C, whereby after such storage the bond strength of an adhesive prepared from such a dental composition to enamel and/or dentin is at least 10 MPa, preferably 15 MPa.

The dental composition may contain from 5 to 90 wt-% of the polymerizable phosphoric acid ester derivative of the present invention.

The present invention will now be explained in further detail by the following examples.

### Example 1

### Ethyl 2-[4-hydroxy-2-oxabutyl]acrylate (1)

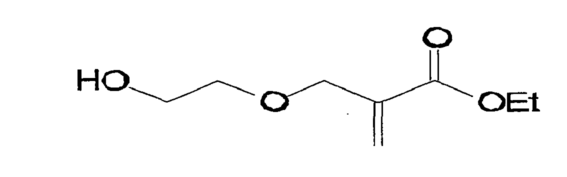

To a solution of 32.52 g trifluoromethanesulphonic anhydride in 100 ml dichloromethane a solution of 15 g (0.115 mol) α-hydroxyethylacrylate and 11.66 g (0.115 mol) triethylamine in 200 ml dichloromethane was added slowly, so that the temperature of the reaction mixture stays below 5 °C. The resulting solution was added drop wise at room temperature to 210 g (1.127 mol) 1,2-ethanediol. After the reaction mixture was stirred for 12 h at room temperature the solution was successively washed with 1 x 200 ml water, 2 x 250 ml of an aqueous sodium carbonate solution (25 wt%) and 1x 200 ml water. The organic layer was dried over magnesium sulphate and filtered. After the evaporation of the solvent the oily raw product was stabilized with 15 mg BHT and purified by vacuum distillation (63 °C/0.032 mbar). This afforded 10.12 g (yield: 50 %) of a clear, colourless oil.
IR(film, cm⁻¹) 3436 (OH), 2979, 2931, 2871 (CH₃/CH₂), 1710 (CO), 1638 (C=C), 1453, 1373, 1304 (CH₃/CH₂), 1270, 1173, 1109, 1052, 953.
¹H-NMR (250 MHz, CDCl₃, ppm) 1.27 (t, 3H, C*H*₃), 2.52 (broad s, 1H, O*H*), 3.54-3.63 (m, 2H, OC*H*₂CH₂O), 3.67-3.78 (m, 2H, OC*H*₂CH₂O), 4.15-4.24 (m, 4H, C*H*₂ (1) and OC*H*₂CH₃), 5.84 (s, 1H, C*H*=C), 6.28 (s, 1H, C*H*=C).
¹³C-NMR (63 MHz, CDCl₃, ppm) 14.06 (CH₃), 60.74 and 61.56 (CH₂ (4) and OCH₂CH₃), 69.31 and 71.80 (CH₂ (1) and CH₂ (3)), 126.21 (C=C-CO), 137.03 (C=C-CO), 165.82 (C=C-CO).

### Ethyl 2-[5-dihydrogen phosphoryl-5,2-dioxapentyl]acrylate (2)

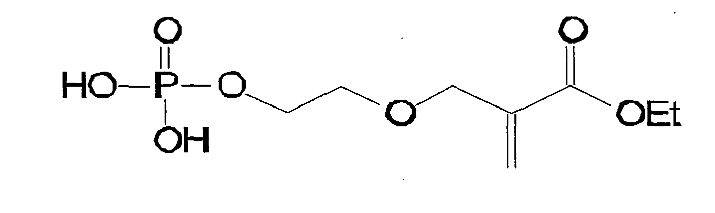

To a stirred solution of 15.46 g (0.1008 mol) phosphorus oxychloride in 280 ml diethyl ether a solution of 17.56 g (0.1008 mol) (1) and 10.2 g (0.1008 mol) triethylamine in 250 ml diethyl ether was added drop wise, while the temperature was kept below 5 °C. After the reaction mixture was stirred for 14 h at room temperature, it was filtered and added slowly at 0 °C to 200 ml water. The emulsion was stirred for 40 min, before the layers were separated and the aqueous layer was washed with 2 x 100 ml diethyl ether. The aqueous layer was narrowed down to 100 ml and extracted with 4 x 100 ml dichloromethane. The organic fractions were united, dried over magnesium sulphate, filtered and evaporated. This yielded 19 g of a yellow oil. The raw product was solved in 400 ml water and washed with 3 x 200 ml diethyl ether. Evaporation of the water at an rotary evaporator and drying under vacuum (10⁻³ mbar) afforded 14.18 g (yield: 55 %) of a clear colourless oil.
IR(film, cm⁻¹) 3500 - 2500 broad absorption (OH), 2912 (CH₃/CH₂), 1709 (CO), 1637 (C=C), 1456, 1374 (CH₃/CH₂), 1261, 1178, 1105, 1014, 949.
¹H-NMR (250 MHz, CDCl₃, ppm) 1.25 (t, 3H, CH₃), 3.70 (broad s, 2H, CH₂), 4.30 - 4.13 (m, 6H, CH₂), 5.87 (s, 1H, C*H*=C), 6.27 (s, 1H, C*H*=C), 10.71 (broad s, 2 H, PO₃*H*₂).
¹³C-NMR (63 MHz, CDCl₃, ppm) 13.94 (*C*H₃), 60.94 (O*C*H₂CH₃), 66.01 (*C*H₂ (4)), 69.25 (*C*H₂ (1)), 69.40 (*C*H₂ (3)), 127.11 (*C*=C-CO), 136.28 (C=*C*-CO), 166.00 (C=C-*C*O).

### Example 2

### Ethyl 2-[12-hydroxy-2-oxadodecyl]acrylate (3)

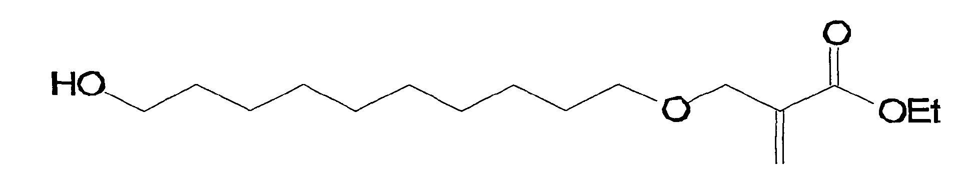

To a solution of 54.7 g trifluoromethanesulphonic anhydride in 210 ml dichloromethane a solution of 25 g (0.192 mol) α-hydroxyethylacrylate and 19.43 g (0.192 mol) triethylamine in 400 ml dichloromethane was added slowly, so that the temperature of the reaction mixture stays below 5 °C. The solution was stirred for 45 min at 0 °C before it was added drop wise at room temperature to a solution of 60 g (0.344 mol) 1,10-decanediol in 400 ml dichloromethane. After the reaction mixture was stirred for 12 h at room temperature the solution was successively washed with 2 x 300 ml of an aqueous sodium carbonate solution (1 n) and 1x400 ml water. The organic layer was dried over magnesium sulphate and filtered. The raw product was purified by column chromatography on silica gel with ethyl acetate as eluens. After the raw product was stabilized with 113 mg BHT, it was finally purified by vacuum distillation (> 150 °C/0.028 mbar). This afforded 11.024 g (yield: 20 %) of a clear, colourless oil.
IR(film, cm⁻¹) 3425 (OH), 2926/2855 (CH₃/CH₂), 1714 (CO), 1638 (C=C), 1459/1375/1303 (CH₃/CH₂), 1270/1172/1102/1031/949.
¹H-NMR (250 MHz, CDCl₃, ppm) 1.08-1.24 (m, 15H, CH₂,CH₃), 1.24-1.49 (m, 4H, CH₂), 3.17 (broad s, 1H, OH), 3.27 (t, 2H, OCH₂) 3.37 (t, 2H, OCH₂), 3.96 (s, 2H, CH₂(1)), 4.01 (q, 2H, OC*H*₂CH₃) 5.65 (s, 1H, C*H*=C), 607 (s, 1H, C*H*=C).
¹³C-NMR (63 MHz, CDCl₃, ppm) 13.64 (*C*H₃), 25.36, 25.68, 28.98, 29.07, 29.11, 19.17 and 32.24 (*C*H₂ (4-11)), 60.10 and 61.90 (*C*H₂ (12) and *C*H₂CH₃), 68.31 and 70.52 (*C*H₂ (1) and *C*H₂ (3)), 124.69 (*C*=C-CO), 137.12 (C=*C*-CO), 165.37 (C=C-*C*O).

### Ethyl 2-[13-dihydrogen phosphoryl-13,2-dioxatridecyl]acrylate (4)

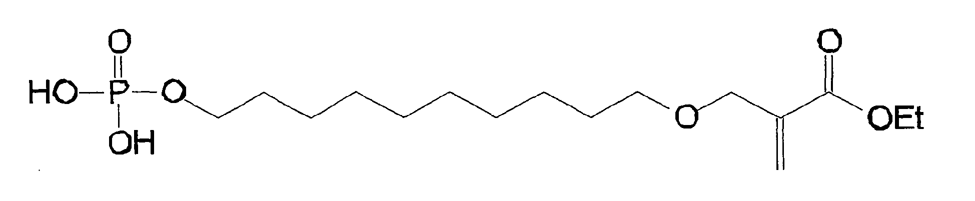

To a stirred solution of 7.082 g (46.18 mmol) phosphorus oxychloride in 120 ml diethyl ether a solution of 11.024 g (38.49 mmol) **(3)** and 4.673 g (46.18 mmol) triethylamine in 150 ml diethyl ether was added drop wise, while the temperature was kept at 0 °C. After the addition was finished the reaction mixture was stirred for 16 h at room temperature before the reaction was finished by filtration of the suspension and evaporation of the solvent. The solution was added drop wise to 300 ml water, while the temperature was kept below 10 °C. After the mixture was stirred for additional 1.5 h at 0 °C, the organic layer was separated and the aqueous fraction extracted with 2 x 100 ml diethyl ether. The organic layers were joined and washed with 5 x 250 ml of an aqueous sodium carbonate solution (25 wt%). The joined aqueous fractions were acidified by the slowly addition of an aqueous hydrochloric acid solution (18 wt%). The acidic solution was washed with 4 x 300 ml diethyl ether. The organic fractions were joined and washed again with 1 x 150 ml water. The separated organic layer was dried over magnesium sulphate, filtered and evaporated. Drying under vacuum (10-3 mbar) afforded 8.57 g (yield: 60 %) of a yellowish solid.
IR(film, cm⁻¹) 3500 - 2500 broad absorption (OH), 2926/2855 (CH₃/CH₂), 1715 (CO), 1639 (C=C), 146111375 (CH₃/CH₂), 1265/1169/1101/1023/951
¹H-NMR (250 MHz, CDCl₃, ppm) 1.15-1.36 (m, 15H, CH₂,CH₃), 1.45-1.77 (m, 4H, CH₂), 3.41 (t, 2H, CH₂(3)), 4.03-4.24 (m, 4H, C*H*₂OPO₃H₂, OC*H*₂CH₃); 4.11 (s, 2H, CH₂(1)), 5.79 (s, 1H, C*H*=C), 6.22 (s, 1H, C*H*=C), 10.74 (broad s, 2H).
¹³C-NMR (63 MHz, CDCl₃, ppm) 13.98 (*C*H₃), 25.08, 25.93, 28.92, 29.19, 29.25, 29.30 and 29.45 (*C*H₂ (4-11)), 60.42 (*C*H₂CH₃), 68.63 and 70.86 (*C*H₂ (1) and *C*H₂ (3)), 125.08 (*C*=C-CO), 137.38 (C=*C*-CO), 165.69 (C=C-*C*O),

### Example 3

### Ethyl 2-[7-hydroxy-2,5-dioxaheptyl]acrylate (5)

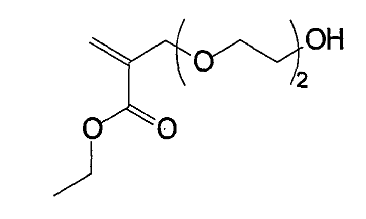

To a solution of 54.7 g (0.1919 mol) trifluoromethanesulphonic anhydride in 200 ml dichloromethane a solution of 25 g (0.192 mol) α-hydroxyethylacrylate and 19.43 g (0.1919 mol) triethylamine in 400 ml dichloromethane was added slowly, so that the temperature of the reaction mixture stays below 5 °C. Afterwards the reaction mixture was stirred for 2 h at 0 °C, before it was added drop wise at room temperature to a solution of 104 g (0.88 mol) diethylene glycol in 400 ml dichloromethane. After the reaction mixture was stirred for 16 h at room temperature the solution was successively washed with 2 x 400 ml of an aqueous sodium carbonate solution (1 n) and 2x 400 ml water. The organic layer was dried over magnesium sulphate and filtered. After the evaporation of the solvent the oily raw product was stabilized with 160 mg BHT and purified by vacuum distillation (94 °C/0.026 mbar). This afforded 19.445 g (yield: 46 %) of a clear, colourless oil.
IR(film, cm⁻¹) 3426 (OH), 2871 (CH₃/CH₂), 1711 (CO), 1639 (C=C), 1456, 1374, 1303 (CH₃/CH₂), 1270, 1174, 1099, 1064, 1028, 952.
¹H-NMR (250 MHz, CDCl₃, ppm) 1.10 (t, 3H, CH₃), 3.29 (s, 1H, OH), 3.36-3.47 (m, 2H, CH₂), 3.47-3.56 (m, 6H, CH₂), 3.97-4.11 (m, 4H, CH₂), 5.69 (s, 1H, C*H*=C), 6.09 (s, 1H, C*H*=C).
¹³C-NMR (63 MHz, CDCl₃, ppm) 13.64 (*C*H₃), 60.19, 61.02, 68.78, 69.76, 69.66, 72.15, 125.22 (*C*=C-CO), 136.71 (C=*C*-CO), 165.28 (C=C-*C*O).

### Ethyl 2-[8-dihydrogen phosphoryl-2,5,8-trioxaoctyl]acrylate (6)

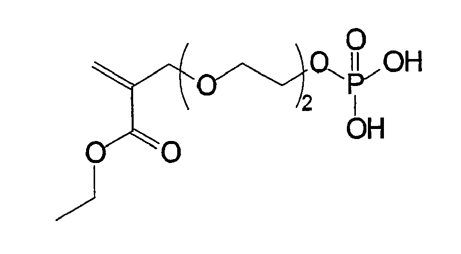

To a stirred solution of 13.27 g (0.086 mol) phosphorus oxychloride in 240 ml diethyl ether a solution of 18.90 g (0.086 mol) **(5)** and 8.76 g (0.086 mol) triethylamine in 220 ml diethyl ether was added drop wise, while the temperature was kept below 5 °C. After the reaction mixture was stirred for 23 h at room temperature, it was filtered and added slowly at 0 °C to 100 ml water. The emulsion was stirred for 1 h, before the layers were separated and the aqueous layer was washed with 4x 100 ml diethyl ether. The aqueous layer was evaporated solved in dichloromethane, dried over magnesium sulphate and filtered. After drying under vacuum (10⁻³ mbar) 17.00 g (yield: 65 %) of a clear colourless oil were isolated.
IR(film, cm⁻¹) 3500 - 2500 broad absorption (OH), 2875 (CH₃/CH₂), 1710 (CO), 1637 (C=C), 1458, 1375 (CH₃/CH₂), 1245, 1176, 1088, 973.
¹H-NMR (250 MHz, CDCl₃, ppm) 1.26 (t, 3H, CH₃), 3.64-3.78 (m, 6H, CH₂), 3.95-4.30 (m, 6H, CH₂), 5.85 (s, 1H, C*H*=C), 6.26 (s, 1H, C*H*=C), 10.53 (s, 2H, OH).
¹³C-NMR (63 MHz, CDCl₃, ppm) 14.00 (*C*H₃), 60.66 (O*C*H₂CH₃). 65.84 (d, *C*H₂ (7)), 69.16, 69.72, 70.06, 70.15, 126.30 (*C*=C-CO), 136.65 (C=*C*-CO). 165.77 (C=C-*C*O).

### Example 4 (Reference)

### (2,2 (4),4)-Trimethylhexamethylene bis(acrylamide)

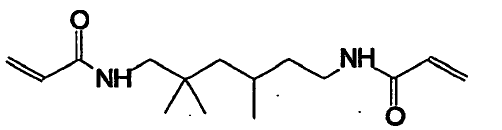

To a solution of 60 g (0.379 mol) (2,2 (4),4)-trimethylhexamethylene diamine in 100 ml methylene chloride a solution of 72.05 g (0.796 mol) acryloyl chloride in 130 ml methylene chloride and a solution of 31.84 g (0.796 mol) sodium hydroxide in 200 ml water were added simultaneously under stirring, so that the temperature remains at 0-5 °C. Thereafter the mixture was stirred at room temperature for additional two hours. The reaction was terminated by the addition of 100 ml water. The organic phase was separated and the aqueous solution was extracted twice with 30 ml methylene chloride. The combined organic liquids were washed with 150 ml of 1 n HCl, 150 ml of 1N NaHCO₃ and with 150 ml water. The solution of the product was stabilized with 0.025 mol% BHT and dried over sodium sulfate. Filtration and evaporation of the solvent yielded 87 g (yield: 86 %) of a colorless, highly viscose oil.
IR(film cm⁻¹) 3411, 3278 (NH), 2957 (CH2/CH3), 1656 (NHCO), 1546 (C=C), 1241 (CH2/CH3), 984/956 (C=C), 703 (C=C).
¹H-NMR (250 MHz, CDCl₃, ppm) 0.7 - 3.4 (several m, 18H, CH₃, CH₂), 5.51-5.60 (m, 1H, CH=C-CO), 6.16-6.26 (m, 2H, CH=CH-CO), 7.12, 6.84, 6.70, 6.59 (4 x t, 2H, NH). ¹³C-NMR (63 MHz, CDCl₃, ppm) 20.80, 22.21, 25.36, 26.01, 26.75, 27.90, 28.02, 29.04, 30.73, 32.80, 35.00, 35.68, 37.21, 38.79, 40.51, 45.16, 46.02, 47.02, 48.70, 125.66 (C=), 131.08 (C=), 165.82 (C=O), 166.09 (C=O).

### Application Example 1 (FBE-02.66.01)

1.058 g N,N'-Bisacrylamido-N,N'-diethyl-1,3-propane, 3.174 g 3,(4),8,(9)-bis(acrylamidomethyl)tricyclo-5.2.1.0^{2,6}decane, 0.602 g Ethyl 2-[13-dihydrogen phosphoryl-13,2-dioxatridecyl]acrylate, 0.03667 g camphor quinone, 0.0878 g bis (2,4,6-trimethylbenzoyl)-phenyl phosphine oxide and 0.04074 g dimethylamino benzoic acid ethyl ester were dissolved in a solvent mixture composed of 3.250 g ethanol and 1.750 g water.

The following procedure was applied for adhesion measurement to enamel and dentin:
- teeth were abraded by 200 and 500 grit abrasive paper
- teeth were stored at 37 °C in water
- treatment with resin formulation: 20 sec
- evaporation by air stream 5 sec
- light curing 20 sec
- Spectrum TPH body cured on tooth 3 times for 20 sec
- Prepared tooth were stored in water at 37 °C for 2h before measured.

Under these conditions the following values were measured adhesion to enamel: 15.2 ± 4.5 MPa, adhesion to dentin: 13.7 ± 2.8 MPa.

### Application Example 2 (FBE-03.71.01)

1.301 g N,N'-Bisacrylamido-N,N`-diethyl-1,3-propane, 1.016 g 3,(4),8,(9)-bis(acrylamidomethyl)tricyclo-5.2.1.0^{2,6}decane, 0.330 g Ethyl 2-[5-dihydrogen phosphoryl-5,2-dioxa-pentyl]acrylate, 0.02203 g camphor quinone, 0.05546 g bis (2,4,6-trimethylbenzoyl)-phenyl phosphine oxide, 0.02561 g dimethylamino benzoic acid ethyl ester and 0.00313 g hydroquinone were dissolved in a solvent mixture composed of 1.463 g ethanol and 0.788 g water.

The following procedure was applied for adhesion measurement to enamel and dentin:
- teeth were abraded by 200 and 500 grit abrasive paper
- teeth were stored at 37 °C in water
- treatment with resin formulation: 20 sec
- evaporation by air stream 5 sec
- light curing 20 sec
- Spectrum TPH body cured on tooth 3 times for 20 sec
- Prepared tooth were stored in water at 37 °C for 2h before measured.

Under these conditions the following values were measured adhesion to dentin: 16.0 ± 3.6 MPa, adhesion to enamel: 10.6 ± 2.8 MPa. After storing the adhesive for 1 week at 50 °C the following values were measured adhesion to dentin: 16.1 ± 1.8 MPa, adhesion to enamel: 12.7 ± 1.7 MPa.

## Claims

1. A polymerizable phosphoric acid ester derivative having the following formula (A): 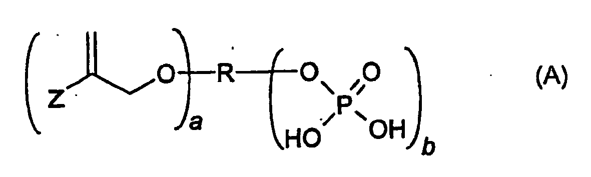 wherein
Z is COOR₁, COSR₁, CON(R₁)₂, CONR₁R₂, or CONHR₁,
R₁ and R₂ independently are
a hydrogen atom, a substituted or unsubstituted C₁ to C₁₈ alkyl group optionally substituted by a C₃ to C₈ cycloalkyl group, a substituted or unsubstituted C₃ to C₈ cycloalkyl group, a substituted or unsubstituted C₄ to C₁₈ aryl or heteroaryl group, a substituted or unsubstituted C₅ to C₁₈ alkylaryl or alkylheteroaryl group, or a substituted or unsubstituted C₇ to C₃₀ aralkyl group, whereby two R₁ residues may form together with the N-atom to which they are bonded a 5- to 7-membered heterocyclic ring which may contain beside said N-atom a further nitrogen atom or an oxygen atom, and whereby the substituted groups may be substituted by 1 to 5 C₁ to C₅ alkyl group(s);
a is an integer of from 1 to 10, preferably 1 to 5;
b is an integer of from 1 to 10, preferably 1 to 5; and
R represents
an (a+b)-valent saturated aliphatic C₂ to C₁₈ group having at least 2 of said primary aliphatic carbon atoms, and optionally 1 or more of said secondary aliphatic carbon atom(s), whereby said (a+b)-valent group may be substituted by C₁ to C₅ alkyl group(s); or
a C₂ to C₄₅ mono-, di-, or polyether which has from 1 to 14 oxygen atoms and is substituted by at least 2 C₁ to C₁₀ aliphatic group(s) having said primary and/or secondary aliphatic carbon atoms; whereby said ether may optionally be substituted by C₁ to C₅ alkyl group(s);
or
wherein the organic residue R represents:
a saturated C₃ to C₈ cyclic, C₇ to C₁₅ bi- or polycyclic hydrocarbon group having from 0 to 4, preferably, 0 to 3, more preferably 0 or 1, of said secondary alicyclic carbon atoms; and/or
a C₄ to C₁₈ aryl or heteroaryl group having from 0 to 5, preferably 0 to 3, more preferably 0 or 1, of said aromatic carbon atoms;
whereby said saturated hydrocarbon or aryl or heteroaryl group is substituted by
from 0 to 5 C₁ to C₅ alkyl group(s);
from 0 to 4, preferably 1 to 3, more preferably 1 or 2, saturated C₁ to C₁₀ aliphatic group(s) having said primary and/or secondary aliphatic carbon atoms, and/or
from 0 to 2 divalent residues according to one of the following formulas:
- [O-CH₂CH₂-]_{f}- wherein f is an integer of from 1 to 10, preferably 1 to 5;
- [-O-CH₂CH₂ CH₂-]_{g}- wherein g is an integer offrom 1 to 10, preferably 1 to 5;
- [O-R₁₂]ₕ- wherein R₁₂ is -CH(CH₃)-CH₂- or -CH₂-CH(CH₃)- and h is an integer of from 1 to 10, preferably 1 to 5;
- [-O-R₁₄]ᵢ-[O-R₁₅]ⱼ- or -[O-R₁₅]ₖ-[O-R₁₄]ₗ- wherein R₁₄ is -CH₂CH₂-, R₁₅ is
- CH(CH₃)-CH₂- or -CH₂-CH(CH₃)-, i, j, k, and l are integers whereby 2i+3j ≤ 15 and 2k+3l ≤ 15,
- [O-CH₂CH₂CH₂CH₂-]ᵣ- wherein r is an integer of 1 or 2;
wherein said divalent residues have one of said primary aliphatic carbon atoms; and
whereby 2 groups selected from said saturated hydrocarbon, aryl, and heteroaryl groups may optionally be linked by a single bond, an alkylene group, or -O-.

2. The polymerizable phosphoric acid ester derivative according to claim 1, wherein the organic residue R is
an (a+b)-valent saturated C₃ to C₈ cyclic or C₇ to C₁₅ bi- or tricyclic hydrocarbon group having at least 2 of said secondary alicyclic carbon atoms;
an (a+b)-valent saturated C₄ to C₁₈ aryl or heteroaryl group having from 2 to 6 of said aromatic carbon atoms;
an (a+b)-valent C₆ to C₁₈ alkylaryl or alkyl heteroaryl group having at least one of said aromatic carbon atoms, at least one of said secondary aliphatic carbon atoms, and optionally one of said primary aliphatic carbon atoms at the terminal end of the alkyl moiety of said alkylaryl or alkylheteroaryl group; or
an (a+b)-valent C₈ to C₃₀ aralkyl group having at least one of said primary aliphatic carbon atoms and at least one of said secondary aliphatic carbon atoms.

3. The polymerizable phosphoric acid ester derivative according to claim 1, **characterized by** one of the following formulas: 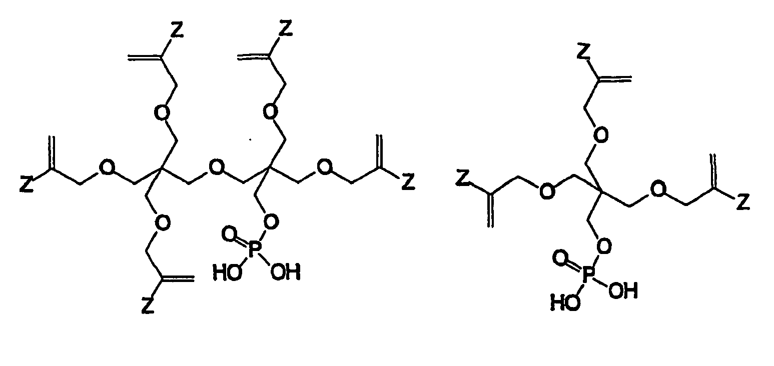 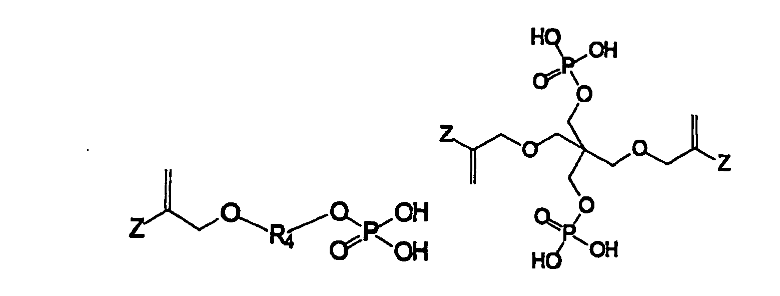 wherein
Z is as defined in claim 1,
R₄ denotes a divalent C₂ to C₁₈ alkylene group, a divalent C₃ to C₈ cycloalkylene group, a divalent C₄ to C₁₈ aryl or heteroaryl group, a divalent C₅ to C₁₈ alkylaryl or alkylheteroaryl group, a divalent C₇ to C₃₀ aralkyl group, whereby each of said groups may be substituted by 1 to 5 C₁ to C₅ alkyl group(s).

4. The polymerizable phosphoric acid ester derivative according to claim 3, wherein R₄ is a divalent residue according to one of the following formulas:
- [CH₂CH₂-O-]ₘ-CH₂CH₂- wherein m is an integer of from 1 to 14,
- [CH₂CH₂ CH₂-O-]ₚ-CH₂CH₂ CH₂- wherein p is an integer of from 1 to 14,
- [R₁₂-O]_{q}-R₁₃- wherein R₁₂ and R₁₃ may be -CH(CH₃)-CH₂- or
- CH₂-CH(CH₃)- and q is from 1 to 14,
- [R₁₄-O]ᵣ-[R₁₅-O]ₛ-R₁₄- or -[R₁₄-O]ₜ-[R₁₅-O]ᵤ-R₁₅- wherein R₁₄ is
- CH₂CH₂-, R₁₅ is -CH(CH₃)-CH₂- or -CH₂-CH(CH₃)-, r, s, t, and u are integers whereby 2r + 3s ≤ 43 and 2t + 3u ≤ 42,
- [CH₂CH₂CH₂CH₂-O-]ᵣCH₂CH₂CH₂CH₂- wherein r is 1 or 2, 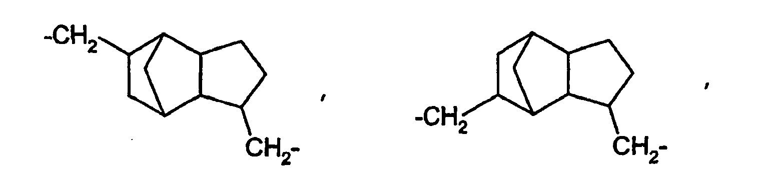 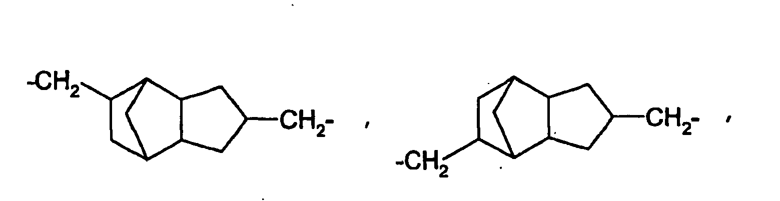 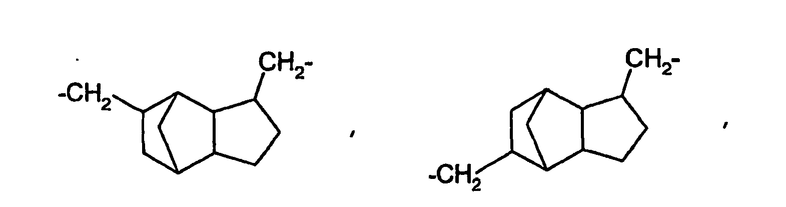 or 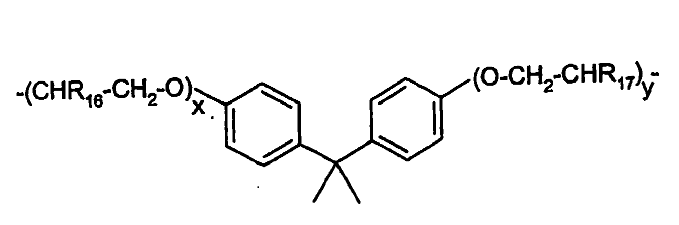 wherein R₁₆ and R₁₇ are H or -CH₃ and x and y may independently be integers of from 0 to 10, preferably 0 to 5.

5. The polymerizable phosphoric acid ester derivative according to any one of claims 1 to 4, wherein said (a+b) carbon atoms are primary aliphatic carbon atoms.

6. The polymerizable phosphoric acid ester derivative according to any one of claims 1 to 5, which is hydrolysis stable under acidic conditions, preferably at a pH of at most 4, more preferably at a pH of at most 2.

7. A method for producing the polymerizable phosphoric acid ester derivative according to any one of claims 1 to 6, which comprises the following steps:
(i) reacting a di- or polyol of the formula (HO)ₐ-R-(OH)_{b} (B) with a compound of the formula (C): 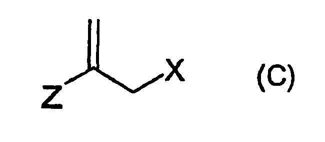 wherein Z, R, a, and b are as defined in any one of claims 1 to 4, particularly wherein R is R₄ as defined in claim 3 or 4, and
X is a leaving group for producing a compound (D) having b hydroxyl group(s) per molecule, and
(ii) reacting compound (D) with a phosphoric acid derivative (E) reactive with a hydroxyl group.

8. The method according to claim 7, wherein the equivalent ratio between compound (C) and the di- or polyol (B) is about a : 1.

9. The method according to claim 7 or 8, wherein the equivalent ratio between compounds (E) and (D) is about b : 1.

10. The method according to any one of claims 7 to 9, wherein the phosphoric acid derivative (E) is phosphorus trichloride oxide, and the method comprises hydrolyzing the reaction product of compounds (D) and (E).

11. The method according to any one of claims 7 to 10, wherein b hydroxyl groups of the polyol (B) are protected, then reacted with compound (C) followed by deprotecting before conducting step (ii).

12. Use of the polymerizable phosphoric acid ester derivative according to any one of claims 1 to 6 in a dental composition.

13. A dental composition comprising the polymerizable phosphoric acid ester derivative of any one of claims 1 to 6.

14. The dental composition of claim 13, which is acidic, preferably it has a pH of at most 4, more preferably a pH of at most 2, most preferably a pH of about 1.0.

15. The dental composition of claim 13 or 14, which further comprises a curing system.

16. The dental composition according to claim 15, wherein the curing system comprises a polymerization initiator, an inhibitor, and a stabilizer.

17. The dental composition according to any one of claims 13 to 16, which further comprises an organic water soluble solvent and/or water.

18. The dental composition according to any one of claims 13 to 17, which further comprises an organic and/or inorganic acid.

19. The dental composition according to any one of claims 13 to 18, which further comprises a polymerizable N-substituted alkylacrylic or acrylic acid amide monomer.

20. The dental composition according to claim 19, wherein the polymerizable N-substituted alkylacrylic or acrylic acid amide monomer is **characterized by** the following formulas: 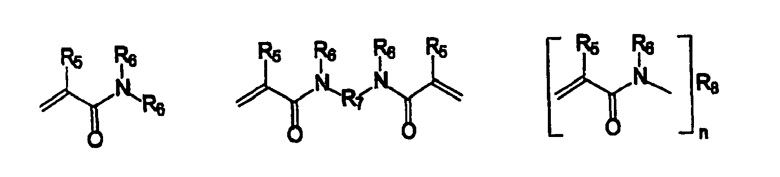 wherein
R₅ and R₆ independently represent an hydrogen atom, a substituted or unsubstituted C₁ to C₁₈ alkyl group, a substituted or unsubstituted C₃ to C₁₈ cycloalkyl group, a substituted or unsubstituted C₄ to C₁₈ aryl or heteroaryl group, a substituted or unsubstituted C₅ to C₁₈ alkylaryl or alkylheteroaryl group, a substituted or unsubstituted C₇ to C₃₀ aralkyl group, whereby two R₆ residues may form together with the N-atom to which they are bonded a 5- to 7-membered heterocyclic ring which may contain beside said N-atom a further nitrogen atom or an oxygen atom; and whereby the substituted groups may be substituted by 1 to 5 C₁ to C₅ alkyl group(s);
R₇ denotes a divalent substituted or unsubstituted organic residue having from 1 to 45 carbon atoms, whereby said organic residue may contain from 1 to 14 oxygen and/or nitrogen atoms and is selected from
a C₁ to C₁₈ alkylene group wherein from 1 to 6 -CH₂-groups may be replaced by a -N-(C=O)-CR₉=CH₂ group wherein R₉ is a hydrogen atom or a C₁ to C₁₈ alkyl group,
a divalent substituted or unsubstituted C₃ to C₁₈ cycloalkyl or cycloalkylene group,
a divalent substituted or unsubstituted C₄ to C₁₈ aryl or heteroaryl group,
a divalent substituted or unsubstituted C₅ to C₁₈ alkylaryl or alkylheteroaryl group,
a divalent substituted or unsubstituted C₇ to C₃₀ aralkyl group, and
a divalent substituted or unsubstituted C₂ to C₄₅ mono-, di- or polyether group having from 1 to 14 oxygen atoms,
R₈ denotes a saturated di- or multivalent substituted or unsubstituted C₂ to C₁₈ hydrocarbon group, a saturated di- or multivalent substituted or unsubstituted cyclic C₃ to C₁₈ hydrocarbon group, a di- or multivalent substituted or unsubstituted C₄ to C₁₈ aryl or heteroaryl group, a di- or multivalent substituted or unsubstituted C₅ to C₁₈ alkylaryl or alkylheteroaryl group, a di- or multivalent substituted or unsubstituted C₇ to C₃₀ aralkyl group, or a di- or multivalent substituted or unsubstituted C₂ to C₄₅ mono-, di-, or polyether residue having from 1 to 14 oxygen atoms,
n is an integer, preferably from 2 to 10, more preferably from 3 to 4.

21. The dental composition according to claim 20, wherein said polymerizable monomer is a mono-, bis- or poly(meth) acrylamide **characterized by** one of the following formulas:

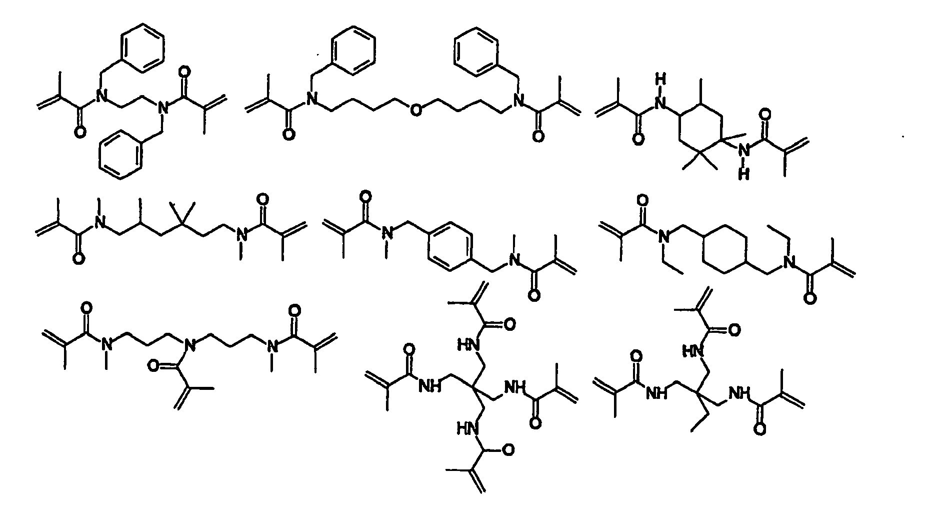

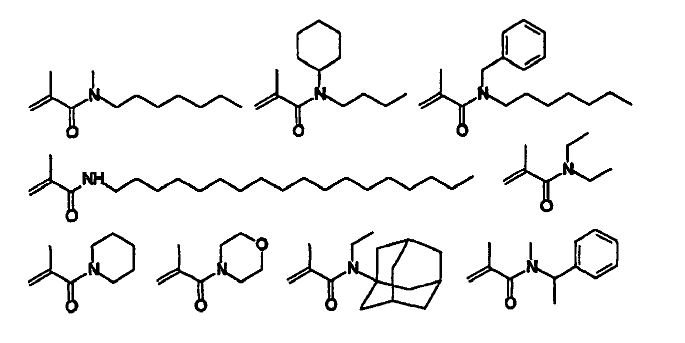

22. The dental composition according to any one of claims 13 to 21, which is a hydrolysis stable one-part self-etching, self-priming dental adhesive composition.

23. The dental composition according to any one of claims 13 to 22, which is hydrolysis stable for at least one week at a storage temperature of 50 °C, whereby after such storage the bond strength of an adhesive prepared from such a dental composition to enamel and/or dentin is at least 10 MPa, preferably 15 MPa.

24. The dental composition according to any one of claims 13 to 23, which contains from 5 to 90 wt-% of the polymerizable phosphoric acid ester derivative according to claim 1.

## Patentansprüche

1. Polymerisierbares Phosphorsäureesterderivat der folgenden Formel (A): 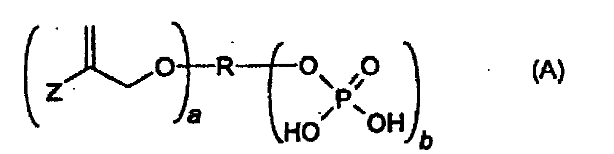 wobei
Z für COOR₁, COSR₁, CON(R₁)₂, CONR₁R₂ oder CONHR₁ steht,
R₁ und R₂ unabhängig bedeuten
ein Wasserstoffatom,
eine substituierte oder unsubstituierte C₁ bis C₁₈ Alkylgruppe, die gegebenenfalls substituiert ist mit einer C₃ bis C₈ Cycloalkylgruppe, einer substituierten oder unsubstituierten C₃ bis C₈ Cycloalkylgruppe, eine substituierte oder unsubstituierte C₄ bis C₁₈ Aryl- oder Heteroarylgruppe,
eine substituierte oder unsubstituierte C₅ bis C₁₈ Alkylaryl- oder Alkylheteroarylgruppe, oder eine substituierte oder unsubstituierte C₇ bis C₃₀ Aralkylgruppe,
wobei zwei R₁-Reste zusammen mit dem N-Atom an das sie gebunden sind, einen 5- bis 7-gliedrigen heterozyklischen Ring bilden können, der neben dem N-Atom ein weiteres Stickstoffatom oder ein Sauerstoffatom enthalten kann, und wobei die substituierten Gruppen substituiert sein können mit 1 bis 5 C₁ bis C₅ Alkylgruppen;
a eine ganze Zahl von 1 bis 10, vorzugsweise 1 bis 5 bedeutet;
b eine ganze Zahl von 1 bis 10, vorzugsweise 1 bis 5 bedeutet; und
R steht für
eine (a+b)-valente gesättigte aliphatische C₂ bis C₁₈ Gruppe steht mit mindestens 2 der primären aliphatischen Kohlenstoffatome, und gegebenenfalls einer oder mehreren der sekundären aliphatischen Kohlenstoffatome, wobei die (a+b)-valente Gruppe mit C₁ bis C₅ Alkylgruppen substituiert sein kann; oder
einen C₂ bis C₄₅ Mono-, Di- oder Polyether, der von 1 bis 14 Kohlenstoffatome aufweist und substituiert ist mit mindestens 2 C₁ bis C₁₀ aliphatischen Gruppen, die die primären und/oder sekundären aliphatischen Kohlenstoffatome aufweisen; wobei der Ether gegebenenfalls substituiert sein kann mit C₁ bis C₅ Alkylgruppen; oder
wobei der organische Rest R steht für:
eine gesättigte C₃ bis C₈ zyklische oder C₇ bis C₁₅ bi- oder
polyzyklische Kohlenwasserstoffgruppe mit 0 bis 4, vorzugsweise 0 bis 3, noch bevorzugter 0 oder 1 der sekundären alizyklischen Kohlenstoffatome; und/oder
eine C₄ bis C₁₈ Aryl- oder Heteroarylgruppe mit 0 bis 5, vorzugsweise 0 bis 3, noch bevorzugter 0 oder 1 der aromatischen Kohlenstoffatome;
wobei die gesättigte Kohlenwasserstoff- oder Aryl- oder Heteroarylgruppe substituiert ist mit
0 bis 5 C₁ bis C₅ Alkylgruppen;
0 bis 4, vorzugsweise 1 bis 3, noch bevorzugter 1 oder 2, gesättigten C₁ bis C₁₀ aliphatischen Gruppen mit den primären und/oder sekundären aliphatischen Kohlenstoffatomen, und/oder 0 bis 2 divalente Reste gemäß einer der folgenden Formeln:
- [O-CH₂CH₂-]_{f}- wobei f für eine ganze Zahl von 1 bis 10 steht, vorzugsweise 1 bis 5;
- [-O-CH₂CH₂CH₂-]_{g}- wobei g für eine ganze Zahl von 1 bis 10, vorzugsweise 1 bis 5 steht;
- [O-R₁₂]ₕ- wobei R₁₂ für -CH(CH₃)-CH₂- oder -CH₂-CH(CH₃)- und h für eine ganze Zahl von 1 bis 10, vorzugsweise 1 bis 5, steht;
- [-O-R₁₄]ᵢ-[O-R₁₅]ⱼ- oder -[O-R₁₅]ₖ-[O-R₁₄]l-steht, wobei R₁₄ für
- CH₂CH₂- steht, R₁₅ für -CH(CH₃)-CH₂- oder -CH₂-CH(CH₃)-steht, i, j, k und l ganze Zahlen sind, wobei 2i + 3j ≤ 15 und 2k +3l ≤ 15 gilt,
- [O-CH₂CH₂CH₂CH₂-]ᵣ- wobei r für eine ganze Zahl von 1 oder 2 steht;
wobei die divalenten Reste ein oder mehrere aliphatische Kohlenstoffatome aufweisen; und
wobei zwei Gruppen, die ausgewählt sind aus diesen gesättigten Kohlenwasserstoff-, Aryl- und Heteroarylgruppen gegebenenfalls verbunden sein können über eine Einfachbindung, eine Alkylengruppe oder -O-.

2. Das polymerisierbare Phosphorsäureesterderivat nach Anspruch 1, wobei der organische Rest R bedeutet
eine (a+b)-valente gesättigte C₃ bis C₈ zyklische oder C₇ bis C₁₅ bi- oder trizyklische Kohlenwasserstoffgruppe mit mindestens zwei der sekundären alizyklischen Kohlenstoffatome;
eine (a+b)-valente gesättigte C₄ bis C₁₈ Aryl- oder Heteroarylgruppe mit 2 bis 6 der aromatischen Kohlenstoffatome;
eine (a+b)-valente C₆ bis C₁₈ Alkylaryl oder Alkylheteroarylgruppe mit mindestens einer der aromatischen Kohlenstoffatome, wobei mindestens eines der sekundären aliphatischen Kohlenstoffatome und gegebenenfalls eines der primären aliphatischen Kohlenstoffatome an einem terminalen Ende der Alkylgruppe der Alkylaryl- oder Alkylheteroarylgruppe vorliegt; oder eine (a+b)-valente C₈ bis C₃₀ Aralkylgruppe mit mindestens einem der primären aliphatischen Kohlenstoffatome und mindesten einem der sekundären aliphatischen Kohlenstoffatome.

3. Das polymerisierbare Phosphorsäureesterderivat nach Anspruch 1, **gekennzeichnet durch** eine der folgenden Formeln: 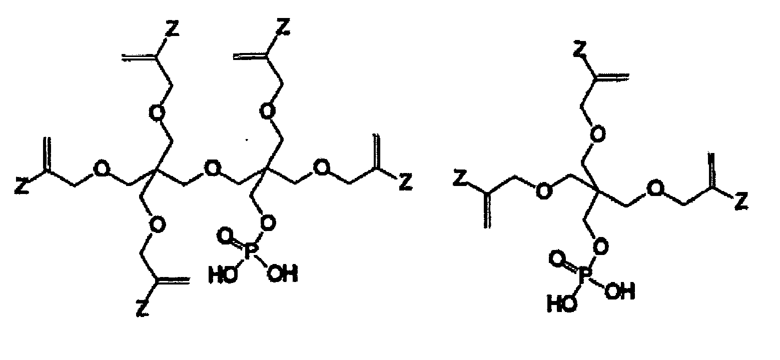 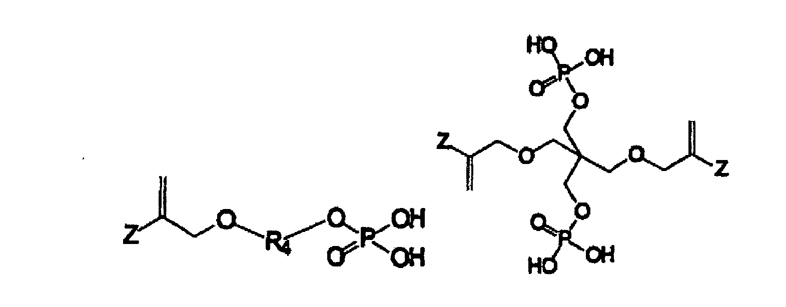 wobei
Z wie in Anspruch 1 definiert ist,
R₄ für eine divalente C₂ bis C₁₈ Alkylengruppe, eine divalente C₃ bis C₈ Cycloalkylengruppe, eine divalente C₄ bis C₁₈ Aryl- oder Heteroarylgruppe, eine divalente C₅ bis C₁₈ Alkylaryl- oder Alkylheteroarylgruppe, eine divalente C₇ bis C₃₀ Aralkylgruppe steht, wobei jede der Gruppen mit 1 bis 5 C₁ bis C₅ Alkylgruppen substituiert sein kann.

4. Das polymerisierbare Phosphorsäureesterderivat nach Anspruch 3, wobei R₄ ein divalenter Rest gemäß einer der folgenden Formeln ist:
- [CH₂CH₂-O-]ₘ-CH₂CH₂- wobei m für eine ganze Zahl von 1 bis 14 steht,
- [CH₂CH₂CH₂-O-]ₚ-CH₂CH₂CH₂- wobei p für eine ganze Zahl von 1 bis 14 steht,
- [R₁₂-O]_{q}-R₁₃- wobei R₁₂ und R₁₃ für -CH(CH₃)-CH₂- oder -CH₂-CH(CH₃)- stehen und q für 1 bis 14 steht,
- [R₁₄-O]ᵣ-[R₁₅-O]ₛ-R₁₄- oder -[R₁₄-O]ₜ-[R₁₅-O]ᵤ-R₁₅-, wobei R₁₄ für -CH₂CH₂-steht, R₁₅ für -CH(CH₃)-CH₂- oder -CH₂-CH(CH₃)- steht, r, s, t und u ganze Zahlen sind, wobei 2r + 3s ≤ 43 und 2t + 3u ≤ 42 gilt,
- [CH₂CH₂CH₂CH₂-O-]ᵣ-CH₂CH₂CH₂CH₂- wobei r für 1 oder 2 steht, 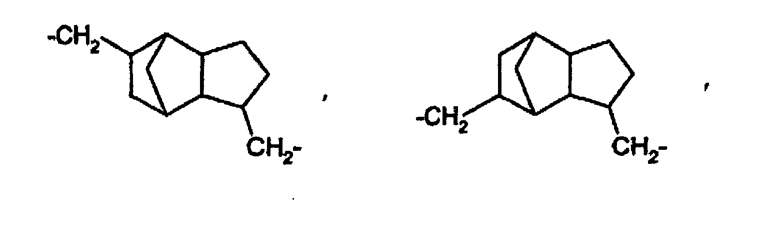 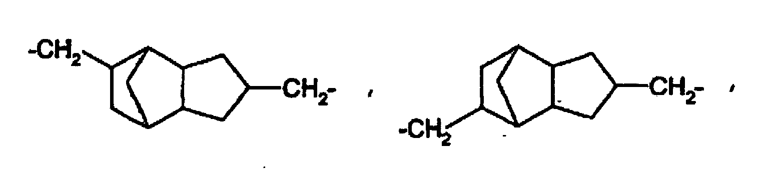 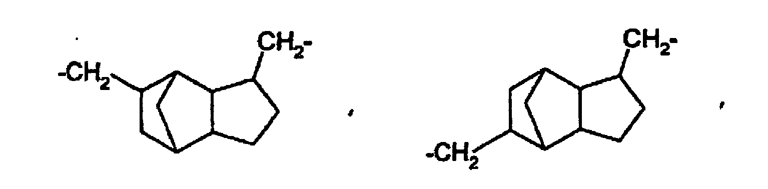 oder 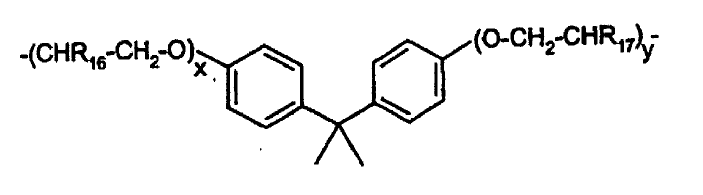 wobei R₁₆ und R₁₇ für H oder -CH₃ stehen und x und y unabhängig voneinander ganze Zahlen von 0 bis 10, vorzugsweise 0 bis 5 bedeuten.

5. Das polymerisierbare Phosphorsäureesterderivat nach einem der Ansprüche 1 bis 4, wobei die (a+b) Kohlenstoffatome primäre aliphatische Kohlenstoffatome sind.

6. Das polymerisierbare Phosphorsäureesterderivat nach einem der Ansprüche 1 bis 5, das unter sauren Bedingungen hydrolysestabil ist, vorzugsweise bei einem pH von höchstens 4, noch mehr bevorzugt bei einem pH von höchstens 2.

7. Verfahren zur Herstellung des polymerisierbaren Phosphorsäureesterderivats nach einem der Ansprüche 1 bis 6, das umfasst die folgenden Stufen:
(i) Umsetzung eines Di- oder Polyols der Formel (HO)ₐ-R-(OH)_{b} (B) mit einer Verbindung der Formel (C): 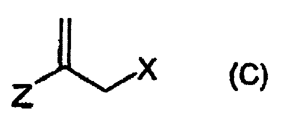 wobei Z, R, a und b wie in einem der Ansprüche 1 bis 4 definiert sind, insbesondere wobei R das gleiche wie R₄ in Anspruch 3 oder 4 bedeutet, und X für eine Abgangsgruppe steht, zur Herstellung einer Verbindung (D), die b Hydroxylgruppen pro Molekül aufweist, und
(ii) Umsetzung der Verbindung (D) mit einem Phosphorsäurederivat (E) das mit einer Hydroxylgruppe reagiert.

8. Das Verfahren nach Anspruch 7, wobei das Äquivalenzverhältnis zwischen Verbindung (C) und dem Di- oder Polyol (B) bei etwa a : 1 liegt.

9. Das Verfahren nach Anspruch 7 oder 8, wobei das Äquivalenzverhältnis zwischen Verbindungen (E) und (D) bei etwa b : 1 liegt.

10. Das Verfahren nach einem der Ansprüche 7 bis 9, wobei das Phosphorsäurederivat (E) Phosphorsäureoxytrichlorid ist und das Verfahren eine Hydrolyse des Reaktionsprodukts der Verbindungen (D) und (E) umfasst.

11. Das Verfahren nach einem der Ansprüche 7 bis 10, wobei b Hydroxylgruppen des Polyols (B) geschützt werden, dann mit Verbindung (C) umgesetzt werden, gefolgt von einem Entschützen vor der Durchführung von Stufe (ii).

12. Verwendung des polymerisierbaren Phosphorsäureesterderivats nach einem der Ansprüche 1 bis 6 in einer Dentalzusammensetzung.

13. Dentalzusammensetzung, umfassend das polymerisierbare Phosphorsäureesterderivat aus einem der Ansprüche 1 bis 6.

14. Die Dentalzusammensetzung nach Anspruch 13, die sauer ist, vorzugsweise einen pH von höchstens 4 aufweist, noch bevorzugter einen pH von höchstens 2, am meisten bevorzugt einen pH von etwa 1,0.

15. Die Dentalzusammensetzung aus Anspruch 13 oder 14, die ferner ein Härtungssystem umfasst.

16. Die Dentalzusammensetzung nach Anspruch 15, wobei das Härtungssystem einen Polymerisationsinitiator, einen Inhibitor und einen Stabilisator umfasst.

17. Die Dentalzusammensetzung nach einem der Ansprüche 13 bis 16, ferner ein organisches wasserlösliches Lösungsmittel und/oder Wasser enthält.

18. Die Dentalzusammensetzung nach einem der Ansprüche 13 bis 17, die ferner eine organische und/oder anorganische Säure umfasst.

19. Die Dentalzusammensetzung nach einem der Ansprüche 13 bis 18, die ferner ein polymerisierbares N-substituiertes Alkylacrylsäure- oder Acrylsäureamidmonomer umfasst.

20. Die Dentalzusammensetzung nach Anspruch 19, wobei das polymerisierbare N-substituierte Alkylacryl- oder Acrylsäureamidmonomer **gekennzeichnet ist durch** die folgenden Formeln: 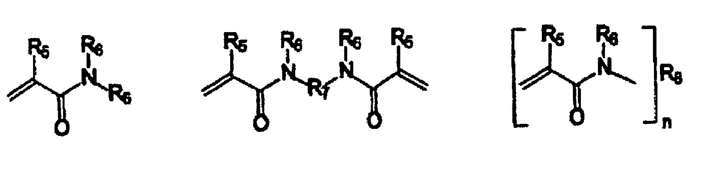 wobei
R₅ und R₆ unabhängig voneinander für ein Wasserstoffatom, eine substituierte oder unsubstituierte C₁ bis C₁₈ Alkylgruppe, eine substituierte C₃ bis C₁₈ Cycloalkylgruppe, eine substituierte oder unsubstituierte C₄ bis C₁₈ Aryl- oder Heteroarylgruppe, eine substituierte oder unsubstituierte C₅ bis C₁₈ Alkylaryl- oder Alkylheteroarylgruppe, eine substituierte oder unsubstituierte C₇ bis C₃₀ Aralkylgruppe stehen, wobei zwei R₆-Reste zusammen mit dem N-Atom an das sie gebunden sind, einen 5- bis 7-gliedrigen heterozyklischen Ring bilden können, der neben den N-Atom enthalten kann ein weiteres Stickstoffatom oder ein Sauerstoffatom; und wobei die substituierten Gruppen mit 1 bis 5 C₁ bis C₅ Alkylgruppen substituiert sein können;
R₇ für einen divalenten substituierten oder unsubstituierten organischen Rest steht, der 1 bis 45 Kohlenstoffatome aufweist, wobei der organische Rest enthalten kann 1 bis 14 Sauerstoff- und/oder Stickstoffatome und ausgewählt aus
einer C₁ bis C₁₈ Alkylengruppe, wobei 1 bis 6 -CH₂-Gruppen ersetzt sein können **durch** eine -N-(C=O)-CR₉=CH₂-Gruppe wobei R₉ für ein Wasserstoffatom oder eine C₁ bis C₁₈ Alkylgruppe steht,
einer divalenten substituierten oder unsubstituierten C₃ bis C₁₈ Cycloalkyl- oder Cycloalkylengruppe,
einer divalenten substituierten oder unsubstituierten C₄ bis C₁₈ Aryl- oder Heteroarylgruppe,
einer divalenten substituierten oder unsubstituierten C₅ bis C₁₈ Alkylaryl- oder Alkyheteroarylgruppe,
einer divalenten substituierten oder unsubstituierten C₇ bis C₃₀ Aralkylgruppe, und
einer divalenten substituierten oder unsubstituierten C₂ bis C₄₅ Mono-, Di- oder Polyethergruppe mit 1 bis 14 Sauerstoffatomen,
R₈ für eine gesättigte di- oder multivalente substituierte oder unsubstituierte C₂ bis C₁₈ Kohlenwasserstoffgruppe, eine gesättigte di- oder multivalente substituierte oder unsubstituierte zyklische C₃ bis C₁₈ Kohlenwasserstoffgruppe, eine di- oder multivalente substituierte oder unsubstituierte C₄ bis C₁₈ Aryl- oder Heteroarylgruppe, eine di- oder multivalente substituierte oder unsubstituierte C₅ bis C₁₈ Alkylaryl- oder Alkylheteroarylgruppe, eine di- oder multivalente substituierte oder unsubstituierte C₇ bis C₃₀ Aralkylgruppe, oder eine di- oder multivalente substituierte oder unsubstituierte C₂ bis C₄₅ Mono-, Di- oder Polyetherrest mit 1 bis 14 Sauerstoffatomen steht,
n für eine ganze Zahl, vorzugsweise von 2 bis 10, noch bevorzugter von 3 bis 4, steht.

21. Die Dentalzusammensetzung nach Anspruch 20, wobei das polymerisierbare Monomer ein Mono-, Bis- oder Poly(meth)acrylamid ist, das **gekennzeichnet ist durch** eine der folgenden Formeln:

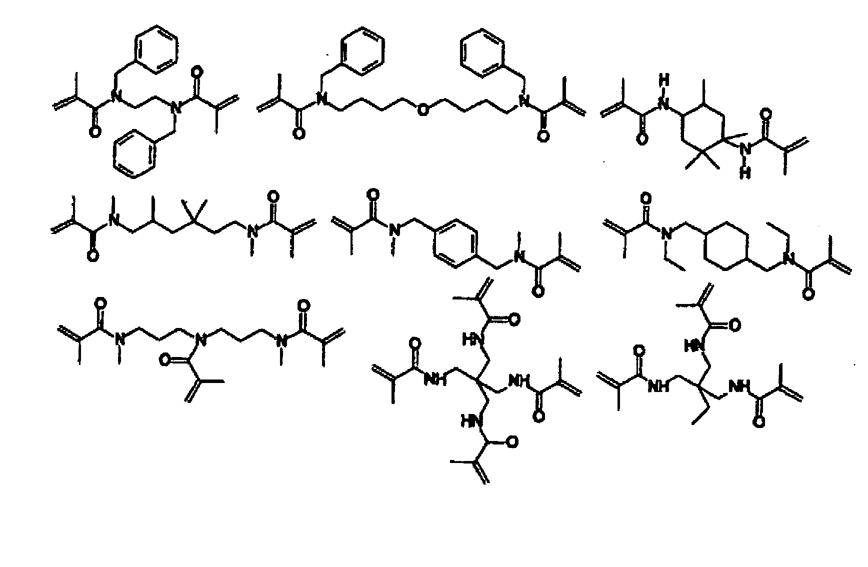

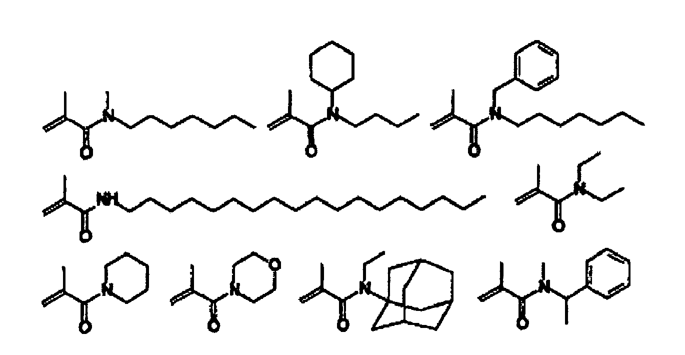

22. Die Dentalzusammensetzung nach einem der Ansprüche 13 bis 21, die eine hydrolysestabile einkomponentige, selbstgrundierende Dentaladhäsivzusammensetzung ist.

23. Die Dentalzusammensetzung nach einem der Ansprüche 13 bis 22, die hydrolysestabil ist für mindestens eine Woche bei einer Lagertemperatur von 50 °C, wobei nach einer solchen Lagerung die Bindungsstärke eines Adhäsivs, das aus der Dentalzusammensetzung erzeugt wird an Schmelz und/oder Dentin mindestens 10 MPa, vorzugsweise 15 MPa, beträgt.

24. Die Dentalzusammensetzung nach einem der Ansprüche 13 bis 23, die 5 bis 90 Gewichtsprozent des polymerisierbaren Phosphorsäureesterderivats nach Anspruch 1 enthält.

## Revendications

1. Dérivé d'ester d'acide phosphorique polymérisable répondant à la formule (A) suivante : 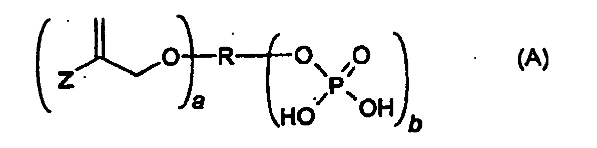 dans laquelle
Z représente un groupe COOR₁, COSR₁, CON (R₁)₂, CONR₁R₂ ou CONHR₁,
R₁ et R₂ représentent indépendamment
un atome d'hydrogène, un groupe alkyle en C₁ à C₁₈, substitué ou non substitué, facultativement substitué avec un groupe cycloalkyle en C₃ à C₈, un groupe cycloalkyle en C₃ à C₈, substitué ou non substitué, un groupe aryle ou hétéroaryle en C₄ à C₁₈ substitué ou non substitué, un groupe alkylaryle ou alkylhétéroaryle en C₅ à C₁₈ substitué ou non substitué ou un groupe aralkyle en C₇ à C₃₀ substitué ou non substitué, deux résidus R₁ pouvant ainsi former, conjointement avec l'atome de N auquel ils sont liés, un noyau hétérocyclique penta- à heptagonal qui peut contenir, en plus dudit atome de N, un atome d'azote supplémentaire ou un atome d'oxygène, et les groupes substitués pouvant ainsi être substitués avec 1 à 5 groupes alkyle en C₁ à C₅ ;
a représente un nombre entier de 1 à 10, de préférence de 1 à 5 ;
b représente un nombre entier de 1 à 10, de préférence de 1 à 5 ; et
R représente
un groupe aliphatique en C₂ à C₁₈ saturé de valence (a+b) comprenant au moins 2 desdits atomes de carbone aliphatiques primaires et facultativement un ou plusieurs desdits atomes de carbone aliphatiques secondaires, ledit groupe de valence (a+b) pouvant ainsi être substitué avec un ou plusieurs groupes alkyle en C₁ à C₅ ; ou
un mono-, di- ou polyéther en C₂ à C₄₅ qui possède 1 à 14 atomes d'oxygène et qui est substitué avec au moins 2 groupes aliphatiques en C₁ à C₁₀ comprenant lesdits atomes de carbone aliphatiques primaires et/ou secondaires ; ledit éther pouvant ainsi être facultativement substitué avec un ou plusieurs groupes alkyle en C₁ à C₅ ;
ou
ledit résidu organique R représente :
un groupe hydrocarboné saturé cyclique en C₃ à C₈, ou bi- ou polycyclique en C₇ à C₁₅ comprenant 0 à 4, avantageusement 0 à 3, plus avantageusement 0 ou 1, desdits atomes de carbone alicycliques secondaires ; et/ou
un groupe aryle ou hétéroaryle en C₄ à C₁₈ comprenant 0 à 5, avantageusement 0 à 3, plus avantageusement 0 ou 1, desdits atomes de carbone aromatiques ;
ledit groupe hydrocarboné saturé ou groupe aryle ou hétéroaryle étant substitué avec
0 à 5 groupes alkyle en C₁ à C₅ ;
0 à 4, avantageusement 1 à 3, plus avantageusement 1 ou 2, groupes aliphatiques en C₁ à C₁₀ saturés comprenant lesdits atomes de carbone aliphatiques primaires et/ou secondaires, et/ou
0 à 2 résidus divalents répondant à une des formules suivantes :
- [O-CH₂-CH₂-]_{f}- dans laquelle f représente un nombre entier de 1 à 10, de préférence de 1 à 5 ;
- [O-CH₂-CH₂-CH₂) _{g}- dans laquelle g représente un nombre entier de 1 à 10, de préférence de 1 à 5 ;
- [O-R₁₂]ₕ-, dans laquelle R₁₂ représente un groupe -CH(CH₃)-CH₂- ou -CH₂-CH(CH₃)- et h représente un nombre entier de 1 à 10, de préférence de 1 à 5 ;
- [-O-R₁₄]ᵢ-[O-R₁₅]ⱼ- ou -[O-R₁₅]ₖ-[O-R₁₄]₁- dans laquelle R₁₄ représente un groupe -CH₂-CH₂-, R₁₅ représente un groupe -CH(CH₃) -CH₂- ou -CH₂-CH(CH₃)-, i, j, k et 1 représentent des nombres entiers satisfaisant les conditions 2i + 3j ≤ 15 et 2k + 31 ≤ 15,
- (O-CH₂CH₂CH₂CH₂-]ᵣ- dans laquelle r représente le nombre entier 1 ou 2 ;
lesdits résidus divalents comprenant un desdits atomes de carbone aliphatiques primaires ; et
2 groupes choisis parmi lesdits groupes hydrocarbonés saturés et groupes aryle et hétéroaryle pouvant être ainsi facultativement liés par une liaison simple, un groupe alkylène ou un groupe -O-.

2. Dérivé d'ester d'acide phosphorique polymérisable suivant la revendication 1, dans lequel le résidu organique R représente
un groupe hydrocarboné saturé cyclique en C₃ à C₈ ou bi- ou tricyclique en C₇ à C₁₅ de valence (a+b) comprenant au moins 2 desdits atomes de carbone alicycliques secondaires ;
un groupe aryle ou hétéroaryle en C₄ à C₁₈ saturé de valence (a+b) comprenant 2 à 6 desdits atomes de carbone aromatiques ;
un groupe alkylaryle ou alkylhétéroaryle en C₆ à C₁₈ de valence (a+b) comprenant au moins un desdits atomes de carbone aromatiques, au moins un desdits atomes de carbone aliphatiques secondaires et facultativement un desdits atomes de carbone aliphatiques primaires à l'extrémité terminale du groupement alkyle dudit groupe alkylaryle ou alkylhétéroaryle ; ou
un groupe aralkyle en C₈ à C₃₀ de valence (a+b) comprenant au moins un desdits atomes de carbone aliphatiques primaires et au moins un desdits atomes de carbone aliphatiques secondaires.

3. Dérivé d'ester d'acide phosphorique polymérisable suivant la revendication 1, **caractérisé par** une des formules suivantes : 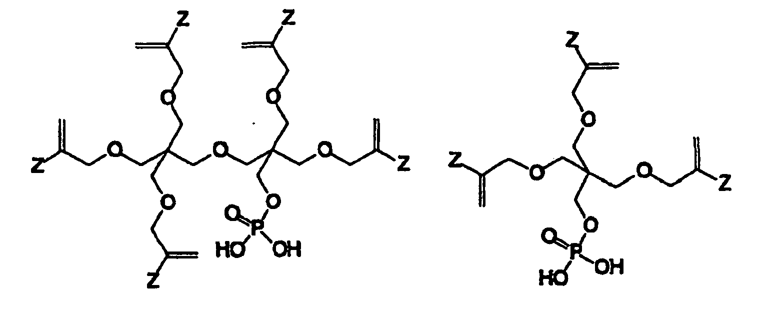 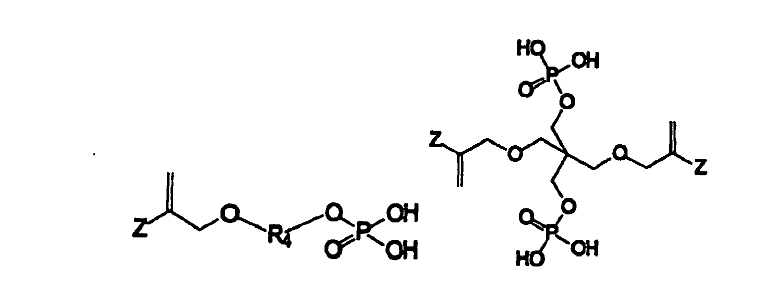 dans lesquelles
Z est tel que défini dans la revendication 1,
R₄ représente un groupe alkylène en C₂ à C₁₈ divalent, un groupe cycloalkyle en C₃ à C₈ divalent, un groupe aryle ou hétéroaryle en C₄ à C₁₈ divalent, un groupe alkylaryle ou alkylhétéroaryle en C₅ à C₁₈ divalent, un groupe aralkyle en C₇ à C₃₀ divalent, chacun desdits groupes pouvant être ainsi substitué avec 1 à 5 groupes alkyle en C₁ à C₅.

4. Dérivé d'ester d'acide phosphorique polymérisable suivant la revendication 3, dans lequel R₄ représente un résidu divalent répondant à une des formules suivantes :
-[CH₂CH₂-O-]ₘ-CH₂CH₂- dans laquelle m représente un nombre entier de 1 à 14,
- [CH₂CH₂CH₂-O-]ₚ-CH₂CH₂CH₂- dans laquelle p représente un nombre entier de 1 à 14,
-[R₁₂-O)_{q}-R₁₃- dans laquelle R₁₂ et R₁₃ peuvent représenter un groupe -CH(CH₃)-CH₂- ou -CH₂-CH(CH₃)- et q a une valeur de 1 à 14 ;
-[R₁₄-O]ᵣ-[R₁₅-O]ₛ-R₁₄- ou -[R₁₄-O]ₜ-[R₁₅-O]ᵤ-R₁₅- dans laquelle R₁₄ représente un groupe -CH₂CH₂-, R₁₅ représente un groupe -CH(CH₃)-CH₂- ou -CH₂-CH(CH₃)-, r, s, t et u représentent les nombres entiers satisfaisant les conditions 2r + 3s ≤ 43 et 2t + 3u ≤ 42,
-[CH₂CH₂CH₂CH₂-O]ᵣ-CH₂CH₂CH₂CH₂- dans laquelle r est égal à 1 ou 2, 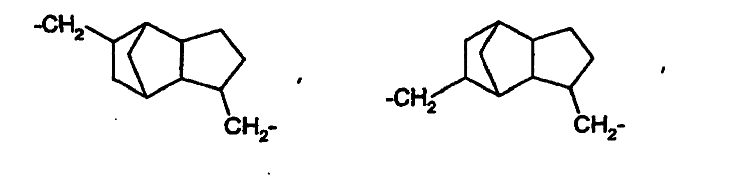 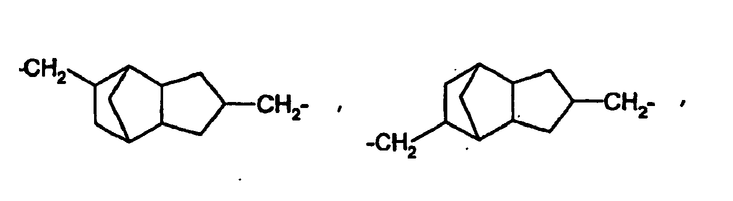 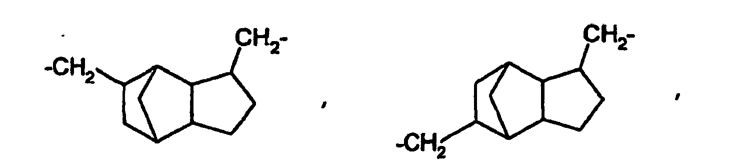 ou 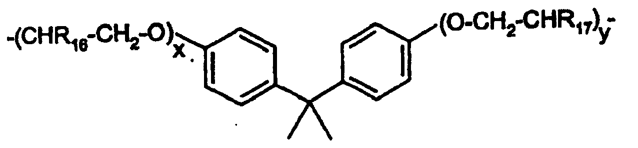 dans laquelle R₁₆ et R₁₇ représentent H ou un groupe -CH₃ et x et y peuvent représenter indépendamment des nombres entiers de 0 à 10, de préférence de 0 à 5.

5. Dérivé d'ester d'acide phosphorique polymérisable suivant l'une quelconque des revendications 1 à 4, dans lequel lesdits (a+b) atomes de carbone sont des atomes de carbone aliphatiques primaires.

6. Dérivé d'ester d'acide phosphorique polymérisable suivant l'une quelconque des revendications 1 à 5, qui est stable à l'hydrolyse dans des conditions acides, avantageusement à un pH d'au plus 4, plus avantageusement à un pH d'au plus 2.

7. Procédé pour la production du dérivé d'ester d'acide phosphorique polymérisable suivant l'une quelconque des revendications 1 à 6, qui comprend les étapes suivantes :
(i) la réaction d'un di- ou polyol de formule (HO)ₐ-R-(OH)_{b} (B) avec un composé de formule (C) : 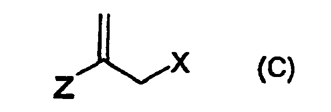 formules dans lesquelles Z, R, a et b sont tels que définis dans l'une quelconque des revendications 1 à 4, en particulier dans lesquelles R représente un groupe R₄ tel que défini dans la revendication 3 ou 4, et X représente un groupe partant, pour la production d'un composé (D) ayant b groupes hydroxyle par molécule, et
(ii) la réaction du composé (D) avec un dérivé d'acide phosphorique (E) réactif avec un groupe hydroxyle.

8. Procédé suivant la revendication 7, dans lequel le rapport des équivalents entre le composé (C) et le di- ou polyol (B) est d'environ a:1.

9. Procédé suivant la revendication 7 ou 8, dans lequel le rapport des équivalents entre les composés (E) et (D) est d'environ b:1.

10. Procédé suivant l'une quelconque des revendications 7 à 9, dans lequel le dérivé d'acide phosphorique (E) est l'oxyde de trichlorure de phosphore, et le procédé comprend l'hydrolyse du produit de réaction des composés (D) et (E).

11. Procédé suivant l'une quelconque des revendications 7 à 10, dans lequel b groupes hydroxyle du polyol (B) sont protégés, puis amenés à réagir avec le composé (C), avec ensuite une suppression de protection avant la mise en oeuvre de l'étape (ii).

12. Utilisation du dérivé d'ester d'acide phosphorique polymérisable suivant l'une quelconque des revendications 1 à 6 dans une composition dentaire.

13. Composition dentaire comprenant le dérivé d'ester d'acide phosphorique polymérisable de l'une quelconque des revendications 1 à 6.

14. Composition dentaire suivant la revendication 13, qui est acide, ayant avantageusement un pH d'au plus 4, plus avantageusement un pH d'au plus 2, de préférence un pH d'environ 1,0.

15. Composition dentaire suivant la revendication 13 ou 14, qui comprend en outre un système de durcissement.

16. Composition dentaire suivant la revendication 15, dans laquelle le système de durcissement comprend un initiateur de polymérisation, un inhibiteur et un stabilisant.

17. Composition dentaire suivant l'une quelconque des revendications 13 à 16, qui comprend en outre un solvant organique hydrosoluble et/ou de l'eau.

18. Composition dentaire suivant l'une quelconque des revendications 13 à 17, qui comprend en outre un acide organique et/ou inorganique.

19. Composition dentaire suivant l'une quelconque des revendications 13 à 18, qui comprend en outre un monomère amide d'acide acrylique ou alkylacrylique N-substitué polymérisable.

20. Composition dentaire suivant la revendication 19, dans laquelle le monomère amide d'acide acrylique ou alkylacrylique N-substitué polymérisable est **caractérisé par** les formules suivantes : 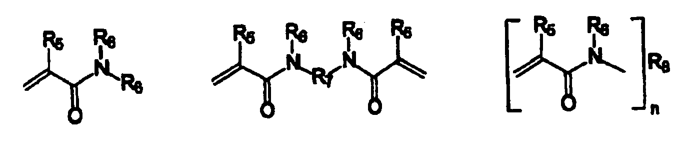 dans lesquelles
R₅ et R₆ représentent indépendamment un atome d'hydrogène, un groupe alkyle en C₁ à C₁₈, substitué ou non substitué, un groupe cycloalkyle en C₃ à C₁₈ substitué ou non substitué, un groupe aryle ou hétéroaryle en C₄ à C₁₈ substitué ou non substitué, un groupe alkylaryle ou alkylhétéroaryle en C₅ à C₁₈ substitué ou non substitué ou un groupe aralkyle en C₇ à C₃₀ substitué ou non substitué, deux résidus R₆ pouvant ainsi former, conjointement avec l'atome de N auquel ils sont liés, un noyau hétérocyclique penta- à heptagonal qui peut contenir, en plus dudit atome de N, un atome d'azote supplémentaire ou un atome d'oxygène, et les groupes substitués pouvant ainsi être substitués avec 1 à 5 groupes alkyle en C₁ à C₅ ;
R₇ représente un résidu organique divalent substitué ou non substitué ayant 1 à 45 atomes de carbone, ledit résidu organique pouvant ainsi contenir 1 à 14 atomes d'oxygène et/ou d'azote et étant choisi entre
un groupe alkylène en C₁ à C₁₈ dans lequel 1 à 6 groupes -CH₂- peuvent être remplacés par un groupe -N-(C=O)-CR₉-CH₂ dans lequel R₉ représente un atome d'hydrogène ou un groupe alkyle en C₁ à C₁₈,
un groupe cycloalkyle ou cycloalkylène en C₃ à C₁₈ divalent substitué ou non substitué,
un groupe aryle ou hétéroaryle en C₄ à C₁₈ divalent substitué ou non substitué,
un groupe alkylaryle ou alkylhétéroaryle en C₅ à C₁₈ divalent substitué ou non substitué,
un groupe aralkyle en C₇ à C₃₀ divalent substitué ou non substitué, et
un groupe mono-, di- ou polyéther en C₂ à C₄₅ divalent substitué ou non substitué comprenant 1 à 14 atomes d'oxygène,
R₈ représente un groupe hydrocarboné en C₂ à C₁₈ saturé di- ou multivalent substitué ou non substitué, un groupe hydrocarboné cyclique en C₃ à C₁₈ saturé di- ou multivalent substitué ou non substitué, un groupe aryle ou hétéroaryle en C₄ à C₁₈ di- ou multivalent substitué ou non substitué, un groupe alkylaryle ou alkylhétéroaryle en C₅ ou C₁₈ di- ou multivalent substitué ou non substitué, un groupe aralkyle en C₇ à C₃₀ di- ou multivalent substitué ou non substitué ou un résidu mono-, di- ou polyéther en C₂ à C₄₅ di- ou multivalent substitué ou non substitué comprenant 1 à 14 atomes d'oxygène,
n représente un nombre entier, avantageusement de 2 à 10, plus avantageusement égal à 3 ou 4.

21. Composition dentaire suivant la revendication 20, dans laquelle ledit monomère polymérisable est un mono-, bis- ou poly(méth)acrylamide **caractérisé par** une des formules suivantes :

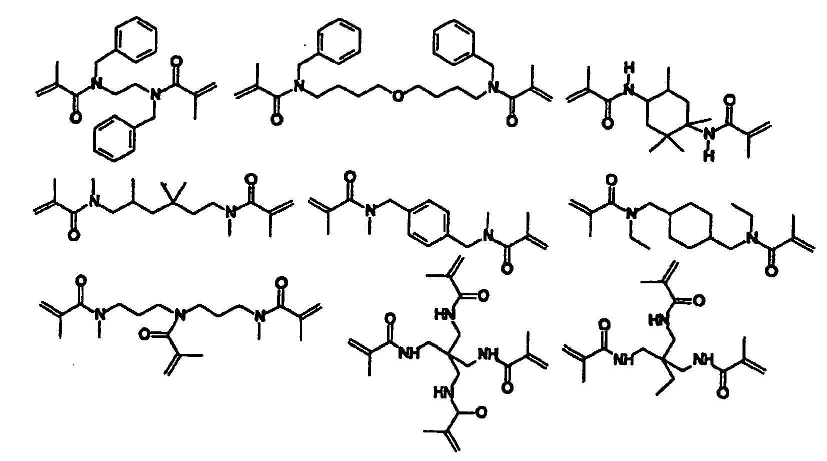

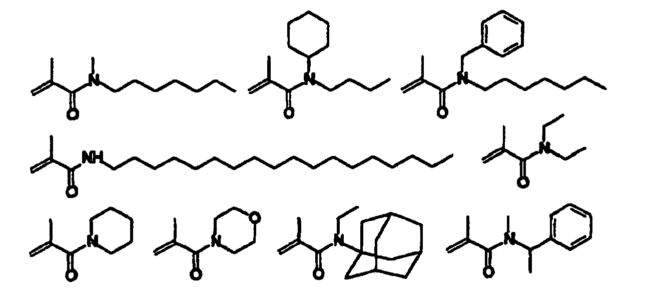

22. Composition dentaire suivant l'une quelconque des revendications 13 à 21, qui est une composition d'adhésif dentaire à auto-corrosion et auto-décapage en une partie, stable à l'hydrolyse.

23. Composition dentaire suivant l'une quelconque des revendications 13 à 22, qui est stable à l'hydrolyse pendant au moins une semaine à une température de stockage de 50°C, la résistance de liaison, après un tel stockage, d'un adhésif préparé à partir d'une telle composition dentaire à l'émail et/ou la dentine étant ainsi égale à au moins 10 MPa, de préférence 15 MPa.

24. Composition dentaire suivant l'une quelconque des revendications 13 à 23, qui contient 5 à 90 % en poids du dérivé d'ester d'acide phosphorique polymérisable suivant la revendication 1.
